(19) Europäisches Patentamt
European Patent Office
Office européen des brevets

(11) **EP 4 085 960 B1**

(12) **EUROPEAN PATENT SPECIFICATION**

(45) Date of publication and mention
of the grant of the patent:
**03.12.2025  Bulletin 2025/49**

(21) Application number: **20909786.4**

(22) Date of filing: **16.12.2020**

(51) International Patent Classification (IPC):
**A61M 16/16** *(2006.01)*     **A61M 16/00** *(2006.01)*
**A61M 16/10** *(2006.01)*

(52) Cooperative Patent Classification (CPC):
**A61M 16/1095; A61M 16/024; A61M 16/16;**
A61M 16/0066; A61M 16/0672; A61M 16/109;
A61M 16/161; A61M 2205/3334; A61M 2205/3368;
A61M 2205/3393

(86) International application number:
**PCT/CN2020/136747**

(87) International publication number:
**WO 2021/135942 (08.07.2021 Gazette 2021/27)**

(54) **HUMIDIFICATION CONTROL SYSTEM AND METHOD FOR VENTILATION THERAPY APPARATUS**

BEFEUCHTUNGSSTEUERUNGSSYSTEM UND -VERFAHREN FÜR VENTILATIONS-THERAPIEGERÄTE

SYSTÈME ET PROCÉDÉ DE COMMANDE D'HUMIDIFICATION POUR UN APPAREIL DE THÉRAPIE DE VENTILATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **31.12.2019  CN 201911424720**

(43) Date of publication of application:
**09.11.2022  Bulletin 2022/45**

(73) Proprietor: **BMC Medical Co., Ltd.**
**Shijingshan**
**Beijing 100041 (CN)**

(72) Inventors:
• **TI, Yao**
  **Beijing 100041 (CN)**
• **ZHUANG, Zhi**
  **Beijing 100041 (CN)**

• **WANG, Qingsong**
  **Beijing 100041 (CN)**
• **ZHANG, Anjun**
  **Beijing 100041 (CN)**

(74) Representative: **Grünecker Patent- und Rechtsanwälte**
**PartG mbB**
**Leopoldstraße 4**
**80802 München (DE)**

(56) References cited:
EP-A1- 4 085 960        WO-A1-2019/190332
CN-A- 104 353 168       CN-A- 105 920 715
CN-A- 107 427 652       CN-A- 108 245 759
CN-A- 110 193 124       CN-A- 111 150 917
US-A1- 2015 165 146     US-A1- 2015 217 079
US-A1- 2016 151 599     US-A1- 2016 151 599

## Description

## TECHNICAL FIELD

The present application relates to the technical field of electronic apparatus, and more particularly, to a humidification control system and method for a ventilation therapy apparatus.

## BACKGROUND

The high-flow-rate warming humidifier is a new respiration treatment apparatus that is capable of humidifying high-flow-rate gas mixed with oxygen and delivering it to the patient. The apparatus may be set according to the actual needs of the patient, to output air of the corresponding flow rate, or to warm and humidify it to the appropriate temperature and humidity after mixing oxygen from the exterior with the ambient air, and delivering it to the nasal cavity of the patient.

US 2015/217079 A1 refers to a humidification breathing apparatus. US 2015/165146 A1 is refers to a positive airway pressure apparatus. US 2016/151599 A1 refers to humidification systems which provide humidified gas. EP 4 085 960 A1 refers to a humidification control system and method.

However, in a period of time after the humidifier is just started up, the water used for humidification has not absorbed sufficient heat yet, therefore the humidifier cannot evaporate sufficient water vapor immediately. In order to ensure the humidity of the air inhaled into the body of the patient, the patient is not prompted to wear the apparatus until the warming and humidification of the humidifier is close to a stable state after a pre-heating process of the started humidifier. However, in practical usage, especially when the patient uses it at home, the patient may use the humidifier in an environment beyond the reasonable environmental condition, and therefore the patient may wear the nasal oxygen cannula before the pre-heating process is completed, which causes the humidity and the temperature of the air inhaled by the patient are not suitable, which not only bring no effect of the treatment, but also may cause damage to the respiratory tract of the patient.

## SUMMARY

The present application provides a humidifier gas-flow control system, to solve the above problems. The invention is defined by the appended claims.

The first aspect of the present application provides a humidification control system of a ventilation-treatment apparatus, wherein the system includes a main ventilation-treatment-apparatus body, a respiration humidifier, a heating pipeline and a nasal oxygen cannula;

the main ventilation-treatment-apparatus body includes a fan and a first controller;

the respiration humidifier includes a water tank, a heating plate, a water-tank-gas-inlet temperature sensor, and a heating-plate temperature sensor;
a gas outlet of the fan is connected to a gas inlet of the water tank;
a gas outlet of the water tank is connected to a gas inlet of the heating pipeline;
a gas outlet of the heating pipeline is connected to an input opening of the nasal oxygen cannula;
the water-tank-gas-inlet temperature sensor is configured for measuring a temperature of the gas inlet of the water tank;
the heating-plate temperature sensor is configured for measuring a temperature of the heating plate;
the heating pipeline includes a gas-inlet temperature sensor of the heating pipe and a heating controlling module of the heating pipeline;
the gas-inlet temperature sensor of the heating pipeline is configured for measuring a temperature of the gas inlet of the heating pipeline;
the first controller is configured for receiving an ambient-air temperature and an ambient-air humidity, a water-tank gas temperature of the water-tank-gas-inlet temperature sensor of the water tank, a heating-plate temperature transmitted by the heating-plate temperature sensor, and a target humidity and a target flow rate that are preset by a user, to determine an air flow rate inputted by the fan, so that a current relative humidity of a first mixed gas flow obtained by mixing water vapor in the water tank and an air inputted by the fan is the target humidity; and
the heating controlling module of the heating pipeline is configured for receiving a temperature of the first mixed gas flow at the gas inlet of the heating pipeline and a temperature of a second mixed gas flow at the gas outlet, to determine a currently required heating power of the heating pipeline, to adjust the heating pipeline to the currently required heating power, to in turn maintain a temperature of a mixed gas flow obtained by mixing the water vapor transmitted via the heating pipeline to the nasal oxygen cannula and the air at a target temperature.

Optionally, the first controller includes an evaporation-rate analyzing module, a flow-rate controlling module and a warming-and-humidification controlling module, and the warming-and-humidification controlling module is configured for, according to the target flow rate, the target humidity, the ambient-air humidity and the ambient-air temperature, determining a heating power of the heating plate, and controlling the heating plate to operate at the heating power;

the evaporation-rate analyzing module is configured for, according to a first time interval, determining a current water evaporation rate in the water tank according to the water-tank gas temperature and the heating-plate temperature; and

the flow-rate controlling module is configured for, according to the current water evaporation rate, adjusting the air flow rate inputted by the fan, so that a relative humidity of the mixed gas flow obtained by mixing the water vapor and the air is the target humidity, till the water evaporation rate in the water tank maintains stable.

Optionally, the operation of the first controller of, according to the target flow rate, the target humidity, the ambient-air humidity and the ambient-air temperature, determining the heating power of the heating plate particularly includes:

by the first controller, according to the target flow rate, the target humidity, the ambient-air humidity and the ambient-air temperature, determining a total water-evaporation amount;
by the first controller, according to the total water-evaporation amount, determining an initial heating power of the heating plate;
by the first controller, determining a heating efficiency of the heating plate according to the ambient-air temperature and a temperature of the heating plate; and
by the first controller, determining the heating power of the heating plate according to the initial heating power of the heating plate and the heating efficiency of the heating plate.

Optionally, the heating controlling module of the heating pipeline controls the heating power of the heating pipeline by:

by the heating controlling module of the heating pipeline, receiving in real time an air flow rate that is inputted by the fan and is sent by the first controller; and
by the heating controlling module of the heating pipeline, determining the heating power of the heating pipeline according to the air flow rate inputted by the fan, the temperature of the first mixed gas flow, the temperature of the second mixed gas flow and the target temperature preset by the user.

Optionally, the operation of, by the heating controlling module of the heating pipeline, according to the air flow rate inputted by the fan, the temperature of the first mixed gas flow, the temperature of the second mixed gas flow and the target temperature preset by the user, determining the heating power of the heating pipeline particularly includes:

by the heating controlling module of the heating pipeline, according to the air flow rate inputted by the fan, the temperature of the first mixed gas flow, the temperature of the second mixed gas flow and the target temperature preset by the user, determining an initial heating power of the heating pipeline;
by the heating controlling module of the heating pipeline, receiving the ambient-air temperature sent by the first controller, and according to the ambient-air temperature and the temperature of the first mixed gas flow, determining the heating efficiency of the heating pipeline; and
by the heating controlling module of the heating pipeline, according to the heating efficiency of the heating pipeline and the initial heating power of the heating pipeline, determining the heating power of the heating pipeline.

Optionally, the first controller includes a first determining module, and the first determining module is configured for, when the water evaporation rate in the water tank maintains stable, determining whether a current air flow rate of the fan is equal to the target flow rate preset by the user; and
when the current air flow rate of the fan is equal to the target flow rate, the first controller controls the heating plate to continue operating at a current heating power and controlling the fan to continue operating at the current air flow rate at the same time.

Optionally, the first controller further includes a second determining module, and the second determining module is configured for, when the current air flow rate of the fan is less than the target flow rate, determining whether the current heating power of the heating plate is a maximum power and being maintained for a second time interval, and when the current heating power of the heating plate is the maximum power and being maintained for the second time interval, the first controller controls the heating plate to continue operating at the current heating power, and simultaneously controls the fan to continue operating at the current air flow rate.

Optionally, when the second determining module determines that the current heating power of the heating plate is not the maximum power, the first controller re-determines the heating power of the heating plate, and simultaneously, according to a water evaporation rate of evaporation when the heating plate operates at the re-determined heating power of the heating plate, adjusts the air flow rate inputted by the fan, till the water evaporation rate maintains stable, to input into the heating pipeline the mixed gas flow obtained by mixing the water vapor and the air, wherein the relative humidity of the mixed gas flow is the target humidity.

Optionally, the system further includes a fan-gas-inlet temperature-and-humidity sensor, and the fan-gas-inlet temperature-and-humidity sensor is configured for measuring a temperature and a humidity at a gas inlet of the fan, to obtain the ambient-air temperature and the ambient-air humidity.

The second aspect of the present application provides a humidification controlling method of a ventilation-treatment apparatus, wherein the method is applied to a humidification control system of a ventilation-treatment apparatus, the humidification control system of a ventilation-treatment apparatus includes a heating plate, a water tank, a fan and a heating pipeline, and the method includes:

Step S1: receiving an ambient-air temperature, an ambient-air humidity and a target temperature and a target flow rate that are preset by a user, and according to the ambient-air temperature, the ambient-air humidity and the target temperature and the target flow rate that are preset by the user, determining a heating power of the heating plate, and controlling the heating plate to operate at the heating power;

Step S2: determining a current water evaporation rate in the water tank;

Step S3: according to the current water evaporation rate in the water tank, determining an air flow rate inputted by the fan, so that a current relative humidity of a first mixed gas flow obtained by mixing a water vapor in the water tank and an air inputted by the fan is the target humidity;

Step S4: according to a third time interval, monitoring a temperature of the first mixed gas flow at a gas inlet of the heating pipeline and a temperature of a second mixed gas flow at a gas outlet of the heating pipeline;

Step S5: according to the temperature of the first mixed gas flow and the temperature of the second mixed gas flow, determining a currently required heating power of the heating pipeline, to adjust the heating pipeline to the currently required heating power, to in turn maintain a temperature of a mixed gas flow obtained by mixing a water vapor transmitted via the heating pipeline to the nasal oxygen cannula and an air at a target temperature;

Step S6: repeating to execute the Step S2 to the Step S3, till the water evaporation rate in the water tank maintains stable; and

Step S7: repeating to execute the Step S4, till the humidification control system of a ventilation-treatment apparatus stops operating.

Optionally, the Step S2 includes:
according to a first time interval, monitoring a water-tank gas temperature and a heating-plate temperature at a water-tank gas inlet, and according to the water-tank gas temperature and the heating-plate temperature, determining the current water evaporation rate in the water tank.

Optionally, the Step S1 includes:

according to the target flow rate, the target humidity, the ambient-air humidity and the ambient-air temperature, determining a total water-evaporation amount;

according to the total water-evaporation amount, determining an initial heating power of the heating plate;

according to the ambient-air temperature and a temperature of the heating plate, determining a heating efficiency of the heating plate; and

according to the initial heating power of the heating plate and the heating efficiency of the heating plate, determining the heating power of the heating plate.

Optionally, the Step S4 includes:
according to a heat dissipation rate of the heating pipeline at a current ambient-air temperature and the temperature of the first mixed gas flow at the gas inlet of the heating pipeline, determining the temperature of the second mixed gas flow at the gas outlet of the heating pipeline.

Optionally, the Step S5 includes:

receiving in real time the air flow rate inputted by the fan;

according to the air flow rate inputted by the fan, the temperature of the first mixed gas flow, the temperature of the second mixed gas flow and the target temperature preset by the user, determining an initial heating power of the heating pipeline;

according to the ambient-air temperature and the temperature of the first mixed gas flow, determining the heating efficiency of the heating pipeline; and

according to the heating efficiency of the heating pipeline and the initial heating power of the heating pipeline, determining the heating power of the heating pipeline.

Optionally, after the Step S6, the method further includes:

when the water evaporation rate in the water tank maintains stable, determining whether a current air flow rate of the fan is equal to the target flow rate preset by the user;

when the current air flow rate of the fan is equal to the target flow rate, controlling the heating plate to continue operating at a current heating power, and simultaneously controlling the fan to continue operating at the current air flow rate;

when the current air flow rate of the fan is less than the target flow rate, determining whether the current heating power of the heating plate is a maximum power and being maintained for a second time interval, and when the current heating power of the heating plate is the maximum power and being maintained for the second time interval, controlling the heating plate to continue operating at a current heating power, and simultaneously controlling the fan to continue operating at the current air flow rate; and

when it is determined that the current heating power of the heating plate is not the maximum power, redetermining the heating power of the heating plate, and simultaneously, according to a water evaporation rate of evaporation when the heating plate operates at the re-determined heating power of the heating plate, adjusting the air flow rate inputted by the fan, till the water evaporation rate maintains stable, to input into the heating pipeline the mixed gas flow obtained by mixing the water vapor and the air, wherein the relative humidity of the mixed gas flow is the target humidity.

As compared with the prior art, the present application has the following advantages:
In the present application, at the pre-heating phase, firstly, by acquiring the ambient-air temperature, the ambient-air humidity and the air temperature inside the water tank, and, by the first controller, receiving the ambient-air temperature, the ambient-air humidity, the gas temperature inside the water tank, and the target flow rate, the target humidity and the target temperature that are preset by the user, and processing them, the present application performs the controlling with the outputted temperature and humidity as the target, and does not perform the controlling with the flow rate as the target, to maintain the outputted gas flow at the suitable humidity at any time. Moreover, by maintaining the temperature of the gas flow inputted into the nasal oxygen cannula by using the heating pipeline, the temperature and the humidity of the gas flow inhaled by the patient may be the target temperature and the target humidity, and when the patient wears the apparatus in advance, the patient will not feel dry. When the humidifier is operating in a condition beyond the environmental condition of normal operation, it may be determined that the preset output may not be reached, and, when the preset output may not be reached, a gas flow of a lower flow rate and a higher humidity may be supplied, rather than a gas flow of a higher flow rate and a lower humidity, which enables the patient to wear more comfortably. The present applicant may ensure that the patient, when wearing the nasal oxygen cannula in advance before the ending of the pre-heating or when the outputting capability of the humidifier is insufficient, may still inhale a gas flow of the suitable temperature and humidity. Accordingly, the present application ensures thoroughly that all of the gas flows inhaled by the patient are the mixed gas flow that satisfies the target temperature and the target humidity, which ensures the comfortableness of the patient.

It should be understood that the above general description and the following detailed description are merely exemplary and explanatory, and may not limit the present disclosure.

The above description is merely a summary of the technical solutions of the present disclosure. In order to more clearly know the elements of the present disclosure to enable the implementation according to the contents of the description, and in order to make the above and other purposes, features and advantages of the present disclosure more apparent and understandable, the particular embodiments of the present disclosure are disposed below.

## BRIEF DESCRIPTION OF THE DRAWINGS

In order to more clearly illustrate the technical solutions of the embodiments of the present disclosure or the prior art, the figures that are required to describe the embodiments or the prior art will be briefly introduced below. Apparently, the figures that are described below are embodiments of the present disclosure, and a person skilled in the art may obtain other figures according to these figures without paying creative work.

In order to more clearly illustrate the technical solutions of the embodiments of the present application, the figures that are required to describe the embodiments of the present application will be briefly introduced below. Apparently, the figures that are described below are embodiments of the present application, and a person skilled in the art may obtain other figures according to these figures without paying creative work.

FIG. 1 is a schematic structural diagram of the humidification control system of a ventilation-treatment apparatus according to the present application;
FIG. 2 is a variation diagram of the water evaporation amount of the humidifier versus the operation period of time according to an embodiment of the present application;
FIG. 3 is a variation diagram of the temperature of the heating plate according to an embodiment of the present application;
FIG. 4 is an operation flow chart of the humidification control system of a ventilation-treatment apparatus according to the present application;
FIG. 5 is a process flow chart of the humidification controlling method of a ventilation-treatment apparatus according to the present application.
FIG. 6 schematically shows a block diagram of a calculating and processing apparatus for implementing the method according to the present disclosure; and
FIG. 7 schematically shows a storage unit for maintaining or carrying a program code for implementing the method according to the present disclosure.

## DETAILED DESCRIPTION OF THE EMBODIMENTS

In order to make the objects, the technical solutions and the advantages of the embodiments of the present disclosure clearer, the technical solutions of the embodiments of the present disclosure will be clearly and completely described below with reference to the drawings of the embodiments of the present disclosure. Apparently, the described embodiments are merely certain embodiments of the present disclosure, rather than all of the embodiments. All of the other embodiments that a person skilled in the art obtains on the basis of the embodiments of the present disclosure without paying creative work fall in the protection scope of the present disclosure.

In order to make the above purposes, features and advantages of the present application more apparent and understandable, the present application will be described in further detail below with reference to the drawings and the particular embodiments.

The primary function of a high-flow-rate warming humidifier is to implement warming and humidification. when the temperature and the humidity of gas outputted by the humidifier are not suitable, the patient inhales the gas may feel very uncomfortable, and even cause damage to his respiratory system.

In accordance with the provisions of Article 101 of the national standard *YY0786-2010 Respiratory Tract Humidifiers for Medical Use-Particular Requirements for Respiratory Humidification Systems,* regarding a humidifying system that is expected to be used for patients of upper-respiratory-tract bypassing, in the prescribed range of the gas flow rate, under the conditions of the setting, the ambient temperature and the outputted gas temperature prescribed in accompanying documents, the outputted gas should be a gas not less than 33mg/L (equivalent to that the temperature is 37°C and the relative humidity is 75%). Furthermore, Article 6 provides that the humidifier is expected to be used for patients of upper-respiratory-tract bypassing, the range and the setting of the regulation on the gas flow rate should be described when the condition that the humidifying system outputs the minimum 33mg/L is satisfied.

However, all of the humidifiers disposed in the related art are pre-heated for a period of time at the beginning of the operation. The humidifier outputs the gas at the preset flow rate, and when the gas is heated to a preset temperature or a temperature lower than the preset temperature by a certain temperature, it is prompted that the pre-heating is ended and the patient may wear the apparatus. For example, the preset temperature is 37°C, and the prompting is made when the actual temperature reaches 34°C (the absolute humidity of saturated steam is 37.5mg/L and exceeds 33mg/L). However, when the humidifier operates in an environmental condition that goes beyond the environmental condition explicitly written in the specification, when the humidifier detects that the temperature and the humidification effect may not reach the demand of the preset temperature of 37°C and humidity of 33mg/L after operating for a period of time, it may merely send an alarm that the target temperature may not be reached, to prompt the patient that the humidifier may not reach the target of output in the current environmental condition. Accordingly, the patient has to make a choice. The patient may feel uncomfortable when he chooses to inhale the presently outputted gas flow, and the therapy may not reach an excellent effect. when the patient chooses not to inhale the presently outputted gas flow, which means that he gives up the therapy, therefore, and the purpose of therapy may not be achieved.

Furthermore, regarding the humidifiers disposed in the related art, due to the power limitation of the humidifiers themselves, the humidifiers may not reach the standard of output of 33mg/L in all conditions. Therefore, the specifications of all of the humidifiers make clear to the ranges of the temperature and the humidity of the environments for normal use, to instruct doctors or patients to use them in reasonable environmental conditions.

In other words, regarding the humidifiers disposed in the related art, in the particular process of usage, there is not always a physician alongside for instruction, or the environment where the humidifiers are located and used may not enable the humidifiers to output a gas not less than 33mg/L (equivalent to that the temperature is 37°C and the relative humidity is 75%). However, the humidifier does not stop operating, or is adaptively adjusted according to the environment where it is located, whereby the outputted gas is still inhaled by the patient, which makes the patient feel extremely uncomfortable, or causes damage to the respiratory system of the patient.

Furthermore, even when the humidifiers disposed in the related art may output a gas not less than 33mg/L (equivalent to that the temperature is 37°C and the relative humidity is 75%), when the gas of a temperature of 37°C and a relative humidity of 75% is inhaled into the body of the patient, the patient may also feel extremely uncomfortable. In practical usage, only when the outputted gas flow reaches 37°C and a relative humidity of 100%, or, in other words, the water vapor contained in the outputted gas is 43.81mg/L, it may ensure that the patient may feel comfortable after inhaling the gas.

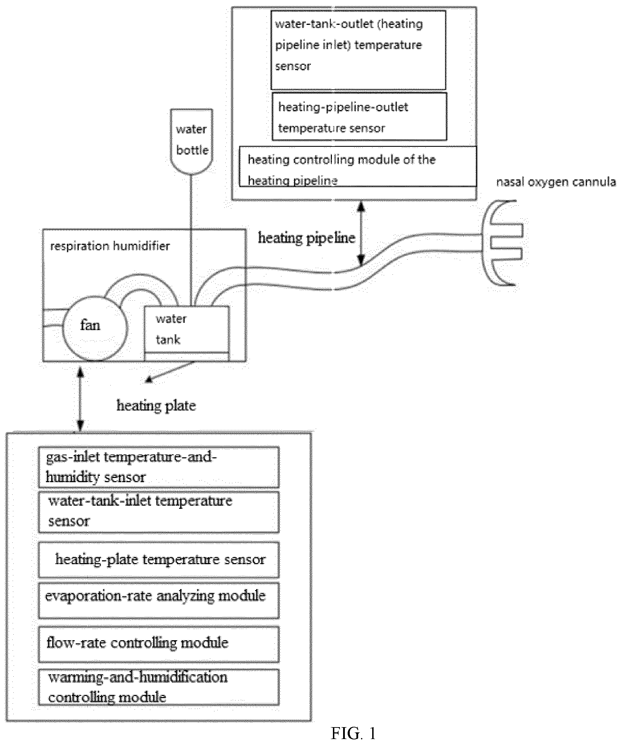

In order to solve the above technical problems, the present application provides a humidification control system of a ventilation-treatment apparatus shown in FIG. 1. The system includes a ventilation-treatment-apparatus main body, a respiration humidifier, a heating pipeline and a nasal oxygen cannula. The ventilation-treatment-apparatus main body includes a fan and a first controller. The respiration humidifier includes a water tank, a heating plate, a water-tank-gas-inlet temperature sensor, and a heating-plate temperature sensor.

The water in the water tank of the respiration humidifier may be from a water bottle. The water bottle is configured to supply water to the respiration humidifier. The water may be a medical liquid, for example, a mixed liquid added with some liquid medicines or a water added with sufficient oxygen, and may also just be water. The respiration humidifier is configured to convert the water dripped from the water bottle into water vapor, whereby the relative humidity of the gas flow flowing through the respiration humidifier reaches 100%. The gas flow flowing out of the respiration humidifier of the relative humidity of 100% is inputted into the heating pipeline. In order to ensure that the temperature of the gas flow outputted from the heating pipeline is the target temperature (the target temperature may be 37°C), the heating pipeline is configured to preserve the temperature of the gas flow flowing through its interior, to enable the temperature of the gas flow inputted into the nasal oxygen cannula by the heating pipeline to be the target temperature.

The fan and the water tank are encapsulated in a housing. A gas outlet of the fan is connected to a gas inlet of the water tank. A gas outlet of the water tank is connected to a gas inlet of the heating pipeline. The water outlet of the water bottle is connected to the water inlet of the water tank. A gas outlet of the heating pipeline is connected to an input opening of the nasal oxygen cannula. The water-tank-gas-inlet temperature sensor is disposed at the gas inlet of the water tank, and is configured to detect the air temperature of the air inputted via the gas inlet of the water tank. The heating-plate temperature sensor is disposed at the heating plate, and is configured to detect the temperature of the heating plate.

The water-tank-gas-inlet temperature sensor and the heating-plate temperature sensor are communicatively connected to the first controller. The first controller is configured for receiving an ambient-air temperature and an ambient-air humidity (the ambient-air temperature and the ambient-air humidity may be obtained by measurement by the fan-gas-inlet temperature-and-humidity sensor, and the fan-gas-inlet temperature-and-humidity sensor may be disposed at the gas inlet of the fan; furthermore, the ambient-air temperature and the ambient-air humidity may also be inputted by the user), a water-tank gas temperature of the water-tank-gas-inlet temperature sensor, a heating-plate temperature transmitted by the heating-plate temperature sensor, and a target humidity and a target flow rate that are preset by a user, to determine an air flow rate inputted by the fan, so that a current relative humidity of a first mixed gas flow obtained by mixing a water vapor in the water tank and air inputted by the fan is the target humidity. The target humidity may be 88%, 90%, 100% and so forth, which may be set according to particular demands.

The mode of generating the mixed gas flow with relative humidity as the target humidity in the water tank is as follows:
The first controller includes an evaporation-rate analyzing module, a flow-rate controlling module and a warming-and-humidification controlling module.

The warming-and-humidification controlling module is configured for, according to the target flow rate, the target humidity, the ambient-air humidity and the ambient-air temperature, determining a heating power of the heating plate, and controlling the heating plate to operate at the heating power.

The water in the water tank is converted into the water vapor because the heating plate heats the water, and the water is evaporated after absorbing the heat to form the water vapor. The fan inputs the ambient air into the water tank, and the ambient air and the water vapor are mixed, to form the mixed gas flow. In order to obtain the mixed gas flow with relative humidity as the target humidity in the water tank, it is necessary to know how much water in the water tank needs to be evaporated, and when the amount of the water to be evaporated is known, it may be calculated how much heat is required to be absorbed to convert that amount of the water into the water vapor, thereby inversely calculating the heating power of the heating plate for the heat required by the water vapor.

In the present application, the amount of the gas flow inhaled by the patient is usually set particularly according to the situation of the patient itself, and the target flow rate required to be inhaled by the patient may be determined accordingly. In order to make the patient feel comfortable, the target temperature and the target humidity of the target flow rate may also be determined. For example, the target temperature is 37°C, and the target humidity is a relative humidity of 100%.

Due to the air inputted by the fan is common atmosphere, the temperature and the humidity of the air inputted by the fan are the atmospheric temperature and the atmospheric humidity, and the air inputted by the fan itself has a certain temperature and a certain humidity accordingly. In the present application, it is required to add the water vapor into the air with a certain temperature and a certain humidity, thereby forming the gas flow which relative humidity is 100%.

The atmospheric temperature and the atmospheric humidity may be obtained by measurement by the fan-gas-inlet temperature-and-humidity sensor, and are referred to as the ambient-air temperature and the ambient-air humidity, respectively.

The warming-and-humidification controlling module, according to the target flow rate, the target humidity, the ambient-air humidity and the ambient-air temperature, may determine the amount of the water required to be evaporated and the heat required to be absorbed by that amount of the water, and then the heating power of the heating plate may be determined, and control the heating plate to operate at the heating power.

Due to the heat generated by the heating plate may not be completely absorbed by the water, and therefore the heat generated by the heating plate involves heat loss. In other words, the heating by the heating plate relates to the issue of heating efficiency. Moreover, the heating efficiency of the heating plate is closely related to the environment, therefore, the actual heating power of the heating plate is obtained in the following manner:
The operation of the first controller of, according to the target flow rate, the target humidity, the ambient-air humidity and the ambient-air temperature, determining the heating power of the heating plate particularly includes:

by the first controller, according to the target flow rate, the target humidity, the ambient-air humidity and the ambient-air temperature, determining a total water-evaporation amount;
by the first controller, according to the total water-evaporation amount, determining an initial heating power of the heating plate;
by the first controller, according to the ambient-air temperature and a temperature of the heating plate, determining a heating efficiency of the heating plate; and

by the first controller, according to the initial heating power of the heating plate and the heating efficiency of the heating plate, determining the heating power of the heating plate.

For example, the ambient-air temperature is 20°C, the ambient-air humidity is 20%, and the current absolute humidity is 3.45mg/L.

The target temperature is 37°C, the target relative humidity is 100%, and the target absolute humidity is 43.81mg/L.

In other words, the absolute humidity is required to be increased by: 43.81mg/L-3.45mg/L=40.36mg/L. (In other words, the water vapor required by per 1L of air is 40.36mg)

Due to the water temperature is equal to the ambient-air temperature, the temperature of the water is 20°C, and the specific enthalpy of water is 83.86kJ/kg.

Moreover, the specific enthalpy of saturated steam at 37 °C is 2567.98kJ/kg. Accordingly, the heat required to be absorbed to convert water into water vapor is:

2567.98kJ/kg-83.86kJ/kg=2484.12kJ/kg; in other words, per 1kg of water should absorb 2484.12kJ from the environment to be converted into water vapor.

Before that, it is known that the water vapor required by per 1L of air is 40.36mg, and accordingly the heat required by 40.36mg of water is: 2484.12kJ/kg*0.00004036kg≈0.100259kJ≈100.26J.

It is set that the target flow rate required by the patient is 40L/min, i.e., 0.67L/s. Moreover, the density of water vapor at 37°C is 0.4kg/m³ ,so the initial heating power required by the heating plate is:

$$\frac{(0.04 kg/m^3)*(0.67L/s)*100.26J}{40.36mg} = 66.7w$$

At the ambient temperature (i.e., the ambient-air temperature), assuming that the heating efficiency of the heating plate at the ambient-air temperature is 70%, then the actual heating power should be 95.5W.

The evaporation-rate analyzing module is configured for, according to a first time interval, according to the water-tank gas temperature and the heating-plate temperature, determining a current water evaporation rate in the water tank.

Generally, the water evaporation rate has relation with the atmospheric pressure, the wind speed and the temperature difference. Because the present application mainly focuses on the evaporation rate of the water in the water tank, merely the influence by the temperature difference on the water evaporation rate is taken into consideration. In the particular usage process, the influence on the water evaporation rate by factors such as the atmospheric pressure and the wind speed may be further taken into consideration according to actual situations.

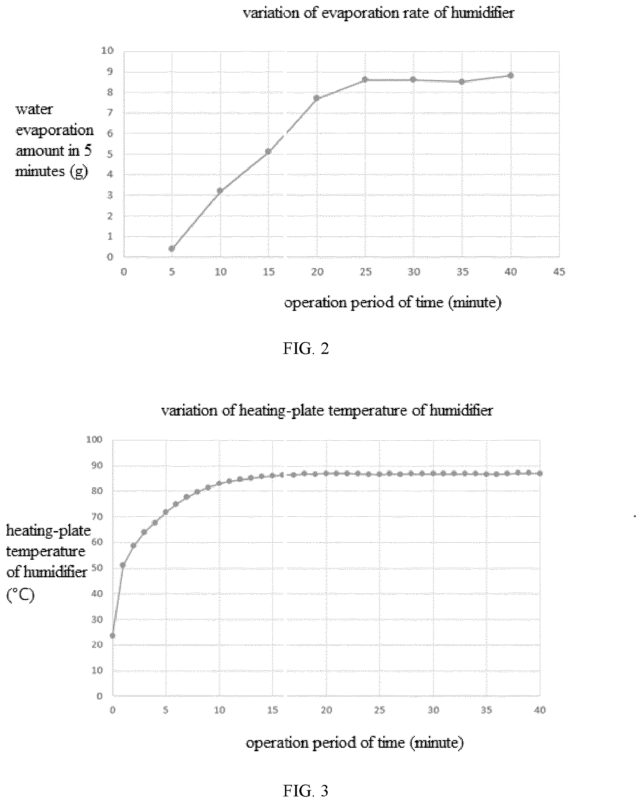

In the present application, the water evaporation rate is measured by means of weighing. For example, as shown in FIGS. 2 and 3, FIGS. 2 and 3 are a variation diagram of the water evaporation amount and a variation diagram of the temperature of the humidifier, respectively, in 40 minutes after a certain time of the operation of the humidifier. In actual operation, after the humidifier starts to operate, the humidifier and the connecting components are weighed one time for every 5 minutes, and the amount of the evaporated water is recorded. It may be seen from FIG. 2 that, in the 20 minutes after the beginning of the operation of the humidifier, the water evaporation amount gradually increases for every 5 minutes, and from the time period of 25th-30th minutes the water evaporation amount reaches a stable state. Moreover, it may be seen from FIG. 3 that the variation of the temperature of the humidifier is similar to that of the evaporation rate, which substantially reaches a stable state at the 15th minute.

Furthermore, due to the heating plate is disposed at the bottom of the water tank, the heating by the heating plate increases the temperature of the air inside the water tank, and also influences the air temperature of the connection between the water tank and the fan to a certain extent. In other words, the vicinity of the pipeline ports connecting the water tank and the fan is influenced by the temperature increase of the air in the water tank to a certain extent, whereby when the air is passing through the connection between the water tank and the fan, the air is slightly heated, therefore, the temperature of the air entering the water tank and the temperature of the air entering the gas inlet of the fan are different. Therefore, the fan-gas-inlet temperature-and-humidity sensor is disposed at the gas inlet of the fan, and is configured to detect the temperature and the humidity of the air entering the fan. Due to the temperature of the gas inlet of the fan and the temperature of the external environment are equal, the temperature and the humidity that are collected by the fan-gas-inlet temperature-and-humidity sensor are used as the ambient-air temperature and the ambient-air humidity. Subsequently, the air temperature at the gas inlet inside the water tank is measured by using the water-tank-gas-inlet temperature sensor.

The flow-rate controlling module is configured for, according to the current water evaporation rate, adjusting the air flow rate inputted by the fan, so that the relative humidity of the mixed gas flow obtained by mixing the water vapor and the air is 100%, till the water evaporation rate in the water tank maintains stable.

After the heating plate starts to operate at the heating power, the temperature of the heating plate starts to increase from normal temperature, and the temperature gradually increases over time. Moreover, the heating power of the heating plate and the stabilized temperature of the heating plate, when the variation of the ambient-air temperature is excluded, which are basically correspond one to one. Therefore, the temperature of the heating plate gradually increases in a certain period of time (referred to as a heating-plate first phase) after the beginning of the heating, and after that, the temperature may be stable (the phase when the temperature is stable is referred to as a heating-plate second phase). As shown in FIG. 3, in the 0-5th minutes, the temperature of the heating plate changes tremendously, with a high warming speed; in the 0-10th minutes, the temperature rising gradually slows down; and after the 10th minute or the 15th minute, the temperature gradually becomes stable.

In the heating-plate first phase, the temperature of the heating plate gradually increases, and, with the increasing of the temperature, the evaporation rate of the water also constantly changes; in other words, the water evaporation amount is also unstable. In order to ensure that the relative humidity of the outputted mixed gas flow in the present application is 100%, it is required to adjust the amount of the air inputted by the fan according to the water evaporation amount, so that when the air inputted by the fan flows out of the gas outlet of the water tank, the relative humidity is 100%.

Therefore, in the heating-plate first phase, it is required to calculate the water evaporation rate periodically, thereby obtaining the water evaporation amount, and adjust the power of the fan according to the water evaporation amount, thereby changing the air amount inputted by the fan.

However, the process of the water absorbing the heat and being converted into the water vapor is lagging as compared with the process of the temperature rising of the heating plate. Therefore, in the heating-plate first phase, in the process of the water absorbing the heat and being converted into the water vapor, the water evaporation rate is unstable, which phase is referred to as a water-evaporation first phase. In the heating-plate second phase, i.e., after the temperature of the heating plate being stable, the water evaporation rate is required to be divided into two phases, which are referred to as a water-evaporation second phase and a water evaporation third phase. The water-evaporation second phase refers to that, although the temperature of the heating plate is already stable, the water evaporation rate is still unstable. After the water-evaporation second phase, the water evaporation third phase is reached, i.e., the phase when the water evaporation rate is stable.

Due to the amount of the air inputted by the fan varies with the water evaporation rate, when the water evaporation amount is stable, the amount of the air inputted by the fan is constant.

All of the heating-plate first phase, the heating-plate second phase, the water-evaporation first phase, the water-evaporation second phase and the water evaporation third phase ensure that the relative humidity of the mixed gas flow obtained by mixing the water vapor and the air brought by the fan is 100%, which makes the patient feel comfortable to a certain extent after inhaling the gas flow. However, to merely ensure the relative humidity of 100% is insufficient, and it is also required to ensure that the temperature of the mixed gas flow is the target temperature. The target temperature is maintained by using the heating pipeline.

The heating pipeline includes a heating-pipe-line-gas-inlet temperature sensor and a heating controlling module of the heating pipeline. The heating-pipe-line-gas-inlet temperature sensor is disposed at the gas inlet of the heating pipeline. The heating-pipe-line-gas-inlet temperature sensor is communicatively connected to the heating controlling module of the heating pipeline.

The heating controlling module of the heating pipeline is configured for receiving a temperature of the first mixed gas flow at the gas inlet of the heating pipeline and a temperature of a second mixed gas flow at the gas outlet, to determine a currently required heating power of the heating pipeline, to adjust the heating pipeline to the currently required heating power, to maintain a temperature of a mixed gas flow obtained by mixing the water vapor transmitted via the heating pipeline to the nasal oxygen cannula and the air at a target temperature.

The temperature of the first mixed gas flow is obtained by measurement by the heating-pipe-line-gas-inlet temperature sensor disposed at the gas inlet of the heating pipeline. The temperature of the second mixed gas flow may be obtained by measurement by a heating-pipeline-gas-outlet temperature sensor disposed at the gas outlet of the heating pipeline, and may also be obtained by calculation according to the temperature of the first mixed gas flow and the heat dissipation rate of the heating pipeline.

When the mixed gas flow outputted from the water tank is directly delivered to the nasal oxygen cannula via a common pipeline without heating or thermal insulation, in an aspect, due to the temperature of the common pipeline is normal temperature but the temperature of the water vapor is higher, the contacting between the water vapor and the common pipeline causes the water vapor to condensate, which reduces the humidity of the mixed gas flow, whereby the humidity of the gas flow inhaled by the patient is insufficient, and it may not ensure that the patient feels comfortable. In another aspect, in the flowing of the mixed gas flow, the heat dissipates, which reduces the temperature. Moreover, the degree of the decreasing of the temperature is closely related to the ambient-air temperature, and, therefore, the temperature of the mixed gas flow when it reaches the nasal oxygen cannula may not be ensured.

In the present application, in order to solve the problem as mentioned above, the heating pipeline is configured to deliver the mixed gas flow. The target temperature of the mixed gas flow delivered in the delivering pipe is 37°C, and, in order to achieve that object, it is required to detect the temperature of the mixed gas flow at the gas inlet of the heating pipeline by using the heating-pipe-line-gas-inlet temperature sensor, detect the temperature of the mixed gas flow at the gas outlet of the heating pipeline by using the heating-pipeline-gas-outlet temperature sensor, and, according to the temperature difference between them and the target temperature, determine the heating power of the heating pipeline.

Further, due to all of the temperatures and the flow rates of the mixed gas flows that are generated at the heating-plate first phase, the heating-plate second phase, the water-evaporation first phase, the water-evaporation second phase and the water evaporation third phase are uncertain, the heating power of the heating pipeline is also uncertain; in other words, the heating power of the heating pipeline is required to be adjusted according to demands.

In order to adjust the heating power of the heating pipeline, it is required to communicatively connect the first controller to the heating controlling module of the heating pipeline. The first controller transmits the target temperature and the air amount inputted by the fan to the heating controlling module of the heating pipeline in real time, and the heating controlling module of the heating pipeline, according to the temperature difference between the temperature of the mixed gas flow at the gas inlet of the heating pipeline and the temperature of the mixed gas flow at the gas outlet of the heating pipeline, the target temperature, and the air amount inputted by the fan, adjusts the heating power of the heating pipeline in real time.

Due to the heating pipeline has the problem of the heating efficiency, the heating controlling module of the heating pipeline controls the heating power of the heating pipeline by:

  by the heating controlling module of the heating pipeline, receiving an air flow rate that is inputted by the fan and is sent by the first controller in real time; and
  by the heating controlling module of the heating pipeline, according to the air flow rate inputted by the fan, the temperature of the first mixed gas flow, the temperature of the second mixed gas flow and the target temperature preset by the user, determining the heating power of the heating pipeline.

Further, the amount of the gas flow flowing through the heating pipeline also influences the degree of the thermal insulation of the gas flow by the heating pipeline. In order to improve the accuracy with which the gas flow at the gas outlet of the heating pipeline is at the target temperature, the operation of, by the heating controlling module of the heating pipeline, according to the air flow rate inputted by the fan, the temperature of the first mixed gas flow, the temperature of the second mixed gas flow and the target temperature preset by the user, determining the heating power of the heating pipeline particularly includes:

  by the heating controlling module of the heating pipeline, according to the air flow rate inputted by the fan, the temperature of the first mixed gas flow, the temperature of the second mixed gas flow and the target temperature preset by the user, determining an initial heating power of the heating pipeline;
  by the heating controlling module of the heating pipeline, receiving the ambient-air temperature sent by the first controller, and according to the ambient-air temperature and the temperature of the first mixed gas flow, determining the heating efficiency of the heating pipeline; and
  by the heating controlling module of the heating pipeline, according to the heating efficiency of the heating pipeline and the initial heating power of the heating pipeline, determining the heating power of the heating pipeline.

As shown in FIG. 2, the evaporation rate that is finally stabilized is 8.6g per 5 minutes and, in average 1.72g per minute, which is 42.6L when divided by 40.36mg/L. In other words, the humidifier may finally stably output a gas flow of a flow rate of 42.6L/min, a temperature of 37°C, and a humidity of 100%.

Comprehensively, the above process may ensure that the temperature of the mixed gas flow outputted from the nasal oxygen cannula is the target temperature, and the humidity of the mixed gas flow is the target humidity. According to particular situations of patients, there are requirements on the amount of the inhaled mixed gas flow, and therefore it is also required to determine whether the flow rate of the mixed gas flow reaches the target flow rate.

In order to solve the above technical problem, in the present application, the first controller includes a first determining module, and the first determining module is configured for, when the water evaporation rate in the water tank maintains stable, determining whether a current air flow rate of the fan is equal to the target flow rate preset by the user; and
when the current air flow rate of the fan is equal to the target flow rate, the first controller controls the heating plate to continue operating at a current heating power, and controls the fan to continue operating at the current air flow rate at the same time.

In other words, when the current air flow rate of the fan is the target flow rate, the humidifier gas-flow control system according to the present application reaches a stable state, and may output the mixed gas flow that satisfies the target flow rate, the target humidity and the target temperature, which may ensure the patient feels comfortable, and may ensure that the patient inhales a sufficient amount of the mixed gas flow.

The first controller further includes a second determining module, and the second determining module is configured for, when the current air flow rate of the fan is less than the target flow rate, determining whether the current heating power of the heating plate is a maximum power and being maintained for a second time interval (the second time interval may be set according to particular situations, for example, 15 minutes and 20 minutes), and when the current heating power of the heating plate is the maximum power and being maintained for the second time interval, the first controller controls the heating plate to continue operating at the current heating power, and controlling the fan to continue operating at the current air flow rate at the same time.

However, in practical usage, the factors of the environment where the humidifier gas-flow control system is used may result in that the current amount of the mixed gas flow is less than the target flow rate, so it is required to determine whether the heating power of the heating plate at the moment is the maximum power of the heating plate. when the heating plate is already operating at the maximum power, then the flow rate of the mixed gas flow outputted currently by the humidifier gas-flow control system is already the maximum discharge that may be outputted in the current environment, therefore, only the temperature and the humidity of the mixed gas flow may be ensured, and the flow rate of the mixed gas flow may not be ensured. At the same time, the patient is prompted that the target flow rate may not be outputted; for example, the target flow rate of 60L/min is set, but a flow rate of 55L/min is finally outputted. In this case, although the humidifier does not reach the preset output, the patient may normally wear it, and may not feel uncomfortable due to insufficient humidity.

When the second determining module determines that the current heating power of the heating plate is not the maximum power, the first controller re-determines the heating power of the heating plate, and at the same time, according to a water evaporation rate of evaporation when the heating plate operates at the re-determined heating power of the heating plate, adjusts the air flow rate inputted by the fan, till the water evaporation rate maintains stable, to input into the heating pipeline the mixed gas flow obtained by mixing the water vapor and the air, wherein the relative humidity of the mixed gas flow is the target humidity.

When the air amount inputted currently by the fan is less than the target flow rate and the heating plate is not operating at the maximum power at the same time, which indicates that the present application is still capable of implementing the target flow rate, so it is required to adjust the heating power of the heating plate, so that the heating power of the heating plate is close to the maximum value or is directly adjusted to be the maximum power, so as to increase the water evaporation rate and increase the water evaporation amount, and thus increase the air amount inputted by the fan, whereby the air amount inputted by the fan is closer to the target flow rate or the air amount inputted by the fan is the target flow rate.

In the present application, at the pre-heating phase, firstly, the present application performs the controlling with the outputted temperature and humidity as the target, and does not perform the controlling with the flow rate as the target, to maintain the outputted gas flow at the suitable temperature and humidity at any time, and when the patient wears the apparatus in advance, the patient will not feel dry. When the humidifier is operating in a condition beyond the environmental condition of normal operation, it may be determined that the preset output may not be reached, and, when the preset output may not be reached, a gas flow of a lower flow rate and a higher humidity may be supplied, rather than a gas flow of a higher flow rate and a lower humidity, which enables the patient to wear more comfortably. The present applicant may ensure that the patient wears the nasal oxygen cannula in advance before the ending of the pre-heating or when the outputting capability of the humidifier is insufficient, so that the patient may still inhale a gas flow of the suitable temperature and humidity. Accordingly, the present application ensures thoroughly that all of the gas flows inhaled by the patient are the mixed gas flow that satisfies the target temperature and the target humidity, which ensures comfort of the patient.

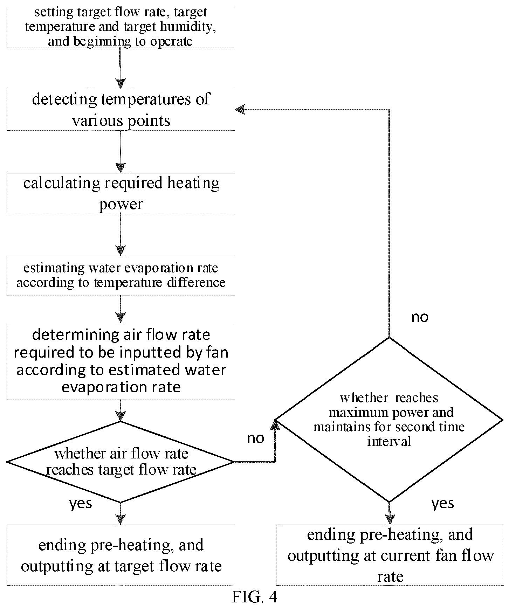

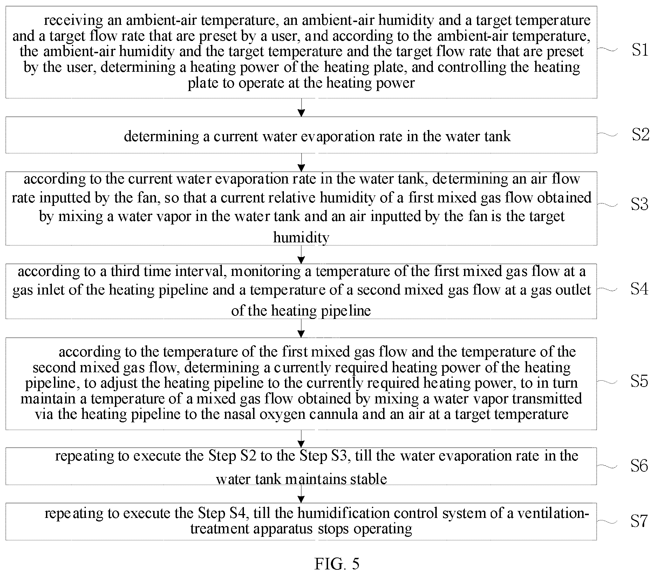

The present application, on the basis of the same technical concept, provides a humidification controlling method of a ventilation-treatment apparatus shown in FIGS. 4 and 5. The method is applied to a humidification control system of a ventilation-treatment apparatus, the humidification control system of a ventilation-treatment apparatus includes a heating plate, a water tank, a fan and a heating pipeline, and the method includes:

Step S1: receiving an ambient-air temperature, an ambient-air humidity and a target temperature and a target flow rate that are preset by a user, and according to the ambient-air temperature, the ambient-air humidity and the target temperature and the target flow rate that are preset by the user, determining a heating power of the heating plate, and controlling the heating plate to operate at the heating power;

Step S2: determining a current water evaporation rate in the water tank;

Step S3: according to the current water evaporation rate in the water tank, determining an air flow rate inputted by the fan, so that a current relative humidity of a first mixed gas flow obtained by mixing a water vapor in the water tank and an air inputted by the fan is the target humidity;

Step S4: according to a third time interval, monitoring a temperature of the first mixed gas flow at a gas inlet of the heating pipeline and a temperature of a second mixed gas flow at a gas outlet of the heating pipeline;

Step S5: according to the temperature of the first mixed gas flow and the temperature of the second mixed gas flow, determining a currently required heating power of the heating pipeline, to adjust the heating pipeline to the currently required heating power, to maintain a temperature of a mixed gas flow obtained by mixing a water vapor transmitted via the heating pipeline to the nasal oxygen cannula and an air at a target temperature;

Step S6: repeating to execute the Step S2 to the Step S3, till the water evaporation rate in the water tank maintains stable; and

Step S7: repeating to execute the Step S4, till the humidification control system of a ventilation-treatment apparatus stops operating.

Particularly, the Step S2 includes:
according to a first time interval, monitoring a water-tank gas temperature and a heating-plate temperature at a water-tank gas inlet, and according to the water-tank gas temperature and the heating-plate temperature, determining the current water evaporation rate in the water tank.

Particularly, the Step S1 includes:

according to the target flow rate, the target humidity, the ambient-air humidity and the ambient-air temperature, determining a total water-evaporation amount;

according to the total water-evaporation amount, determining an initial heating power of the heating plate;

according to the ambient-air temperature and a temperature of the heating plate, determining a heating efficiency of the heating plate; and

according to the initial heating power of the heating plate and the heating efficiency of the heating plate, determining the heating power of the heating plate.

Particularly, the Step S4 includes:
according to a heat dissipation rate of the heating pipeline at a current ambient-air temperature and the temperature of the first mixed gas flow at the gas inlet of the heating pipeline, determining the temperature of the second mixed gas flow at the gas outlet of the heating pipeline.

Particularly, the Step S5 includes:

receiving the air flow rate inputted by the fan in real time;

according to the air flow rate inputted by the fan, the temperature of the first mixed gas flow, the temperature of the second mixed gas flow and the target temperature preset by the user, determining an initial heating power of the heating pipeline;

according to the ambient-air temperature and the temperature of the first mixed gas flow, determining the heating efficiency of the heating pipeline; and

according to the heating efficiency of the heating pipeline and the initial heating power of the heating pipeline, determining the heating power of the heating pipeline.

Particularly, after the Step S6, the method further includes:

when the water evaporation rate in the water tank maintains stable, determining whether a current air flow rate of the fan is equal to the target flow rate preset by the user;

when the current air flow rate of the fan is equal to the target flow rate, controlling the heating plate to continue operating at a current heating power, and controlling the fan to continue operating at the current air flow rate at the same time;

when the current air flow rate of the fan is less than the target flow rate, determining whether the current heating power of the heating plate is a maximum power and being maintained for a second time interval, and when the current heating power of the heating plate is the maximum power and being maintained for the second time interval, controlling the heating plate to continue operating at a current heating power, and controlling the fan to continue operating at the current air flow rate at the same time; and

when determining that the current heating power of the heating plate is not the maximum power, re-determining the heating power of the heating plate, and at the same time, according to a water evaporation rate of evaporation when the heating plate operates at the re-determined heating power of the heating plate, adjusting the air flow rate inputted by the fan, till the water evaporation rate maintains stable, to input into the heating pipeline the mixed gas flow obtained by mixing the water vapor and the air, wherein the relative humidity of the mixed gas flow is the target humidity.

Regarding the process embodiments, because they are substantially similar to the system embodiments, they are described simply, and the related parts may refer to the description on the process embodiments.

The embodiments of the description are described in the mode of progression, each of the embodiments emphatically describes the differences from the other embodiments, and the same or similar parts of the embodiments may refer to each other.

The humidification control system and method of the ventilation-treatment apparatus according to the present application has been described in detail above. The principle and the embodiments of the present application are described herein with reference to the particular examples, and the description of the above embodiments is merely intended to facilitate to understand the method according to the present application and its core concept. Moreover, for a person skilled in the art, according to the concept of the present application, the particular embodiments and the range of application may be varied. In conclusion, the contents of the description should not be understood as limiting the present application.

The above-described apparatus embodiments are merely illustrative, wherein the units that are described as separate components may or may not be physically separate, and the components that are displayed as units may or may not be physical units; in other words, they may be located at the same one location, and may also be distributed to a plurality of network units. Some or all of the modules may be selected according to the actual demands to implement the purposes of the solutions of the embodiments. A person skilled in the art may understand and implement the technical solutions without paying creative work.

Each component embodiment of the present disclosure may be implemented by hardware, or by software modules that are operated on one or more processors, or by a combination thereof. A person skilled in the art should understand that some or all of the functions of some or all of the components of the calculating and processing apparatus according to the embodiments of the present disclosure may be implemented by using a microprocessor or a digital signal processor (DSP) in practice. The present disclosure may also be implemented as apparatus or apparatus programs (for example, computer programs and computer program products) for implementing part of or the whole of the method described herein. Such programs for implementing the present disclosure may be stored in a computer-readable medium, or may be in the form of one or more signals. Such signals may be downloaded from an Internet website, or disposed on a carrier signal, or disposed in any other forms.

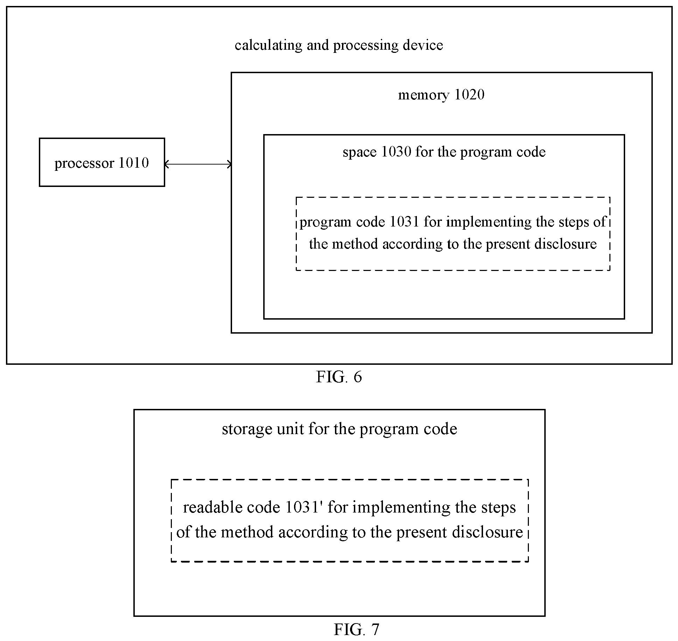

For example, FIG. 6 shows a calculating and processing apparatus that may implement the method according to the present disclosure. The calculating and processing apparatus traditionally includes a processor 1010 and a computer program product or computer-readable medium in the form of a memory 1020. The memory 1020 may be electronic memories such as flash memory, EEPROM (Electrically Erasable Programmable Read Only Memory), EPROM, hard disk or ROM. The memory 1020 has the storage space 1030 of the program code 1031 for implementing any steps of the above method. For example, the storage space 1030 for program code may contain program codes 1031 for individually implementing each of the steps of the above method. Those program codes may be read from one or more computer program products or be written into the one or more computer program products. Those computer program products include program code carriers such as a hard disk, a compact disk (CD), a memory card or a floppy disk. Such computer program products are usually portable or fixed storage units as shown in FIG. 7. The storage unit may have storage segments or storage spaces with similar arrangement to the memory 1020 of the calculating and processing apparatus in FIG. 6. The program codes may, for example, be compressed in a suitable form. Generally, the storage unit contains a computer-readable code 1031', which may be read by a processor like 1010. When those codes are executed by the calculating and processing apparatus, the codes cause the calculating and processing apparatus to implement each of the steps of the method described above.

The "one embodiment", "an embodiment" or "one or more embodiments" as used herein means that particular features, structures or characteristics described with reference to an embodiment are included in at least one embodiment of the present disclosure. Moreover, it should be noted that here an example using the wording "in an embodiment" does not necessarily refer to the same one embodiment.

The description disposed herein describes many concrete details. However, it may be understood that the embodiments of the present disclosure may be implemented without those concrete details. In some of the embodiments, well-known processes, structures and techniques are not described in detail, so as not to affect the understanding of the description.

In the claims, any reference signs between parentheses should not be construed as limiting the claims. The word "include" does not exclude elements or steps that are not listed in the claims. The word "a" or "an" preceding an element does not exclude the existing of a plurality of such elements. The present disclosure may be implemented by means of hardware comprising several different elements and by means of a properly programmed computer. In unit claims that list several apparatuses, some of those apparatus may be embodied by the same item of hardware. The words first, second, third and so on do not denote any order. Those words may be interpreted as names.

Finally, it should be noted that the above embodiments are merely intended to explain the technical solutions of the present disclosure, and not to limit them. Although the present disclosure is explained in detail with reference to the above embodiments, a person skilled in the art should understand that he may still modify the technical solutions set forth by the above embodiments, or make equivalent substitutions to part of the technical features of them. However, those modifications or substitutions do not make the essence of the corresponding technical solutions depart from the scope of the technical solutions of the embodiments of the present disclosure.

## Claims

1. A humidification controlling system of a ventilation-treatment apparatus, wherein the system comprises a ventilation-treatment-device body, a respiration humidifier, a heating pipeline and a nasal oxygen cannula;

   the ventilation-treatment-device body comprises a fan and a first controller;
   the respiration humidifier comprises a water tank, a heating plate, a gas-inlet temperature sensor of the water-tank, and a heating-plate temperature sensor; wherein a gas outlet of the fan is connected to a gas inlet of the water tank; a gas outlet of the water tank is connected to a gas inlet of the heating pipeline; a gas outlet of the heating pipeline is connected to an input opening of the nasal oxygen cannula; the gas-inlet temperature sensor of the water tank is configured for measuring a temperature of the gas inlet of the water tank; the heating-plate temperature sensor is configured for measuring a temperature of the heating plate;

the heating pipeline comprises a gas-inlet temperature sensor of the heating pipeline and a heating controlling module of the heating pipeline; wherein the gas-inlet temperature sensor of the heating pipeline is configured for measuring a temperature of the gas inlet of the heating pipeline;

the first controller is configured for receiving an ambient-air temperature and an ambient-air humidity, a water-tank gas temperature of the gas-inlet temperature sensor of the water tank, a heating-plate temperature transmitted by the heating-plate temperature sensor, and a target humidity and a target flow rate that are preset by a user, to determine an air flow rate inputted by the fan, so that a current relative humidity of a first mixed gas flow obtained by mixing water vapor in the water tank and air inputted by the fan is the target humidity; and

the heating controlling module of the heating pipeline is configured for receiving a temperature of the first mixed gas flow at the gas inlet of the heating pipeline and a temperature of a second mixed gas flow at the gas outlet, to determine a currently required heating power of the heating pipeline, to adjust the heating pipeline to the currently required heating power, to maintain a temperature of a mixed gas flow obtained by mixing the water vapor transmitted via the heating pipeline to the nasal oxygen cannula and the air at a target temperature.

2. The system according to claim 1, **characterized in that**, the first controller comprises an evaporation-rate analyzing module, a flow-rate controlling module and a warming-and-humidification controlling module, and the warming-and-humidification controlling module is configured for, according to the target flow rate, the target humidity, the ambient-air humidity and the ambient-air temperature, determining a heating power of the heating plate, and controlling the heating plate to operate at the heating power;

the evaporation-rate analyzing module is configured for, according to a first time interval, determining a current water evaporation rate in the water tank according to the water-tank gas temperature and the heating-plate temperature; and
the flow-rate controlling module is configured for adjusting the air flow rate inputted by the fan according to the current water evaporation rate, so that a relative humidity of the mixed gas flow obtained by mixing the water vapor and the air is the target humidity, until the water evaporation rate in the water tank maintains stable.

3. The system according to any one of claims 1-2, **characterized in that**, the operation of the first controller of, according to the target flow rate, the target humidity, the ambient-air humidity and the ambient-air temperature, determining the heating power of the heating plate particularly comprises:

by the first controller, determining a total water-evaporation amount according to the target flow rate, the target humidity, the ambient-air humidity and the ambient-air temperature;
by the first controller, determining an initial heating power of the heating plate according to the total water-evaporation amount;
by the first controller, determining a heating efficiency of the heating plate according to the ambient-air temperature and a temperature of the heating plate; and
by the first controller, determining the heating power of the heating plate according to the initial heating power of the heating plate and the heating efficiency of the heating plate.

4. The system according to any one of claims 2-3, **characterized in that**, the way for the heating controlling module of the heating pipeline to control the heating power of the heating pipeline is:

by the heating controlling module of the heating pipeline, receiving an air flow rate that is inputted by the fan and is sent by the first controller in real time; and
by the heating controlling module of the heating pipeline, determining the heating power of the heating pipeline according to the air flow rate inputted by the fan, the temperature of the first mixed gas flow, the temperature of the second mixed gas flow and the target temperature preset by the user.

5. The system according to claim 4, **characterized in that**, the operation of, by the heating controlling module of the heating pipeline, determining the heating power of the heating pipeline according to the airflow rate inputted by the fan, the temperature of the first mixed gas flow, the temperature of the second mixed gas flow and the target temperature preset by the user particularly comprises:

by the heating controlling module of the heating pipeline, determining an initial heating power of the heating pipeline according to the air flow rate inputted by the fan, the temperature of the first mixed gas flow, the temperature of the second mixed gas flow and the target temperature preset by the user;

by the heating controlling module of the heating pipeline, receiving the ambient-air temperature sent by the first controller, and according to the ambient-air temperature and the temperature of the first mixed gas flow, determining the heating efficiency of the heating pipeline; and

by the heating controlling module of the heating pipeline, determining the heating power of the heating pipeline according to the heating efficiency of the heating pipeline and the initial heating power of the heating pipeline.

6. The system according to any one of claims 1-5, **characterized in that**, the first controller comprises a first determining module, and the first determining module is configured for, when the water evaporation rate in the water tank maintains stable, determining whether a current air flow rate of the fan is equal to the target flow rate preset by the user; and

when the current air flow rate of the fan is equal to the target flow rate, the first controller controlling the heating plate to continue operating at a current heating power, and controlling the fan to continue operating at the current air flow rate at the same time.

7. The system according to claim 6, **characterized in that**, the first controller further comprises a second determining module, and the second determining module is configured for, when the current air flow rate of the fan is less than the target flow rate, determining whether the current heating power of the heating plate is a maximum power and being maintained for a second time interval, and when the current heating power of the heating plate is the maximum power and being maintained for the second time interval, the first controller controlling the heating plate to continue operating at the current heating power, and controlling the fan to continue operating at the current air flow rate at the same time.

8. The system according to claim 7, **characterized in that**, when the second determining module determines that the current heating power of the heating plate is not the maximum power, the first controller re-determines the heating power of the heating plate, and at the same time, according to a water evaporation rate of evaporation when the heating plate operates at the re-determined heating power of the heating plate, adjusts the air flow rate inputted by the fan, until the water evaporation rate maintains stable, to input the mixed gas flow obtained by mixing the water vapor and the air into the heating pipeline, wherein the relative humidity of the mixed gas flow is the target humidity.

9. A humidification controlling method of a ventilation-treatment apparatus, wherein the method is applied to a humidification controlling system of a ventilation-treatment apparatus, the humidification controlling system of a ventilation-treatment apparatus comprises a heating plate, a water tank, a fan and a heating pipeline, and the method comprises:

Step S1: receiving an ambient-air temperature, an ambient-air humidity and a target temperature and a target flow rate that are preset by a user, and according to the ambient-air temperature, the ambient-air humidity and the target temperature and the target flow rate that are preset by the user, determining a heating power of the heating plate, and controlling the heating plate to operate at the heating power;

Step S2: determining a current water evaporation rate in the water tank;

Step S3: according to the current water evaporation rate in the water tank, determining an air flow rate inputted by the fan, so that a current relative humidity of a first mixed gas flow obtained by mixing a water vapor in the water tank and an air inputted by the fan is the target humidity;

Step S4: according to a third time interval, monitoring a temperature of the first mixed gas flow at a gas inlet of the heating pipeline and a temperature of a second mixed gas flow at a gas outlet of the heating pipeline;

Step S5: according to the temperature of the first mixed gas flow and the temperature of the second mixed gas flow, determining a currently required heating power of the heating pipeline, to adjust the heating pipeline to the currently required heating power, to maintain a temperature of a mixed gas flow obtained by mixing water vapor transmitted via the heating pipeline to the nasal oxygen cannula and air at a target temperature;

Step S6: repeating to execute the Step S2 to the Step S3, until the water evaporation rate in the water tank maintains stable; and

Step S7: repeating to execute the Step S4, until the humidification controlling system of a ventilation-treatment apparatus stops operating.

10. The method according to claim 9, **characterized in that**, the Step S2 comprises:
according to a first time interval, monitoring a water-tank gas temperature and a heating-plate temperature at a water-tank gas inlet, and according to the water-tank gas temperature and the heating-plate temperature, determining the current water evaporation rate in the water tank.

11. The method according to any one of claims 9-10, wherein the Step S1 comprises:

determining a total water-evaporation amount according to the target flow rate, the target humidity, the ambient-air humidity and the ambient-air temperature;
determining an initial heating power of the heating plate according to the total water-evaporation amount;
determining a heating efficiency of the heating plate according to the ambient-air temperature and a temperature of the heating plate; and
determining the heating power of the heating plate according to the initial heating power of the heating plate and the heating efficiency of the heating plate.

12. The method according to any one of claims 9-11, **characterized in that**, the Step S4 comprises:
according to a heat dissipation rate of the heating pipeline at a current ambient-air temperature and the temperature of the first mixed gas flow at the gas inlet of the heating pipeline, determining the temperature of the second mixed gas flow at the gas outlet of the heating pipeline.

13. The method according to any one of claims 9-12, **characterized in that**, the Step S5 comprises:

receiving the air flow rate inputted by the fan in real time;
determining an initial heating power of the heating pipeline, according to the air flow rate inputted by the fan, the temperature of the first mixed gas flow, the temperature of the second mixed gas flow and the target temperature preset by the user;
determining the heating efficiency of the heating pipeline according to the ambient-air temperature and the temperature of the first mixed gas flow; and
determining the heating power of the heating pipeline according to the heating efficiency of the heating pipeline and the initial heating power of the heating pipeline.

14. The method according to any one of claims 9-13, **characterized in that**, after the Step S6, the method further comprises:

when the water evaporation rate in the water tank maintains stable, determining whether a current air flow rate of the fan is equal to the target flow rate preset by the user;
when the current air flow rate of the fan is equal to the target flow rate, controlling the heating plate to continue operating at a current heating power, and controlling the fan to continue operating at the current air flow rate at the same time;
when the current air flow rate of the fan is less than the target flow rate, determining whether the current heating power of the heating plate is a maximum power and being maintained for a second time interval, and when the current heating power of the heating plate is the maximum power and being maintained for the second time interval, controlling the heating plate to continue operating at a current heating power, and controlling the fan to continue operating at the current air flow rate at the same time; and
when determining that the current heating power of the heating plate is not the maximum power, re-determining the heating power of the heating plate, and at the same time, according to a water evaporation rate of evaporation when the heating plate operates at the re-determined heating power of the heating plate, adjusting the air flow rate inputted by the fan, until the water evaporation rate maintains stable, to input the mixed gas flow obtained by mixing the water vapor and the air into the heating pipeline, wherein the relative humidity of the mixed gas flow is the target humidity.

**Patentansprüche**

1. Ein Steuersystem für die Befeuchtung einer Beatmungsvorrichtung, wobei das System einen Beatmungsvorrichtungskörper, einen Atemluftbefeuchter, eine Heizleitung und eine nasale Sauerstoffkanüle aufweist;

das Gehäuse der Beatmungsvorrichtung einen Ventilator und eine erste Steuereinheit aufweist; der Atemluftbefeuchter weist einen Wassertank, eine Heizplatte, einen Gaseinlasstemperatursensor des Wassertanks und einen Heizplattentemperatursensor auf; wobei ein Gasauslass des Ventilators mit einem Gaseinlass des Wassertanks verbunden ist; ein Gasauslass des Wassertanks mit einem Gaseinlass der Heizleitung verbunden ist; ein Gasauslass der Heizungsrohrleitung mit einer Eingangsöffnung der nasalen Sauerstoffkanüle verbunden ist; der Gaseinlasstemperatursensor des Wassertanks zum Messen einer Temperatur des Gaseinlasses des Wassertanks konfiguriert ist; der Heizplattentemperatursensor zum Messen einer Temperatur der Heizplatte konfiguriert ist; die Heizungsrohrleitung einen Gaseinlasstemperatursensor der Heizungsrohrleitung und ein Heizungssteuerungsmodul der Heizungsrohrleitung aufweist; wobei der Gaseinlasstemperatursensor der Heizungsrohrleitung zum Messen einer Temperatur des Gaseinlasses der Heizungsrohrleitung ausgebildet ist;

die erste Steuereinheit ist so konfiguriert, dass sie eine Umgebungslufttemperatur und eine Umgebungsluftfeuchtigkeit, eine Wassertank-Gastemperatur des Gaseinlasstemperatursensors des Wassertanks, eine von dem Heizplattentemperatursensor übertragene Heizplattentemperatur und eine Zielfeuchtigkeit und eine Zielströmungsrate, die von einem Benutzer voreingestellt sind, empfängt, um eine von dem Ventilator eingegebene Luftströmungsrate zu bestimmen, so dass eine aktuelle relative Feuchtigkeit einer ersten gemischten Gasströmung, die durch Mischen von Wasserdampf in dem Wassertank und von dem Ventilator eingegebener Luft erhalten wird, die Zielfeuchtigkeit ist; und

das Heizungssteuerungsmodul der Heizungsleitung so konfiguriert ist, dass es eine Temperatur des ersten gemischten Gasstroms am Gaseinlass der Heizungsleitung und eine Temperatur eines zweiten gemischten Gasstroms am Gasauslass empfängt, um eine gegenwärtig erforderliche Heizleistung der Heizungsleitung zu bestimmen, um die Heizungsleitung auf die gegenwärtig erforderliche Heizleistung einzustellen, um eine Temperatur eines gemischten Gasstroms, der durch Mischen des über die Heizungsleitung zur nasalen Sauerstoffkanüle übertragenen Wasserdampfs und der Luft erhalten wird, auf einer Zieltemperatur zu halten.

2. Das System nach Anspruch 1, **dadurch gekennzeichnet, dass** die erste Steuereinheit ein Verdampfungsraten-Analysemodul, ein Durchflussraten-Steuermodul und ein Erwärmungs- und Befeuchtungs-Steuermodul aufweist, und dass das Erwärmungs- und Befeuchtungs-Steuermodul so konfiguriert ist, dass es in Abhängigkeit von der Ziel-Durchflussrate, der Ziel-Feuchtigkeit, der Umgebungs-Luft-Feuchtigkeit und der Umgebungs-Luft-Temperatur eine Heizleistung der Heizplatte bestimmt und die Heizplatte so steuert, dass sie mit der Heizleistung arbeitet;

die Gasanalyseeinheit so konfiguriert ist, dass sie gemäß einem ersten Zeitintervall eine aktuelle Wasserverdampfungsrate in dem Wassertank gemäß der Wassertankgastemperatur und der Heizplattentemperatur bestimmt; und die Steuereinheit zur Steuerung der Durchflussrate so konfiguriert ist, dass sie die durch den Ventilator zugeführte Luftdurchflussrate entsprechend der aktuellen Wasserverdampfungsrate einstellt, so dass eine relative Feuchtigkeit des gemischten Gasstroms, der durch Mischen des Wasserdampfs und der Luft erhalten wird, die Zielfeuchtigkeit ist, bis die Wasserverdampfungsrate im Wassertank stabil bleibt.

3. Das System nach einem der Ansprüche 1 bis 2, **dadurch gekennzeichnet, dass** der Betrieb der ersten Steuereinheit zur Bestimmung der Heizleistung der Heizplatte in Abhängigkeit von der Soll-Durchflussmenge, der Soll-Feuchte, der Umgebungsluftfeuchte und der Umgebungslufttemperatur insbesondere aufweisend ist:

durch die erste Steuereinheit die Bestimmung einer Gesamt-Wasserverdunstungsmenge in Abhängigkeit von der Soll-Durchflussmenge, der Soll-Luftfeuchtigkeit, der Umgebungs-Luftfeuchtigkeit und der Umgebungs-Lufttemperatur;
Bestimmen einer anfänglichen Heizleistung der Heizplatte durch die erste Steuereinheit in Abhängigkeit von der Gesamtwasserverdunstungsmenge;
durch die erste Steuereinheit, Bestimmen einer Heizleistung der Heizplatte entsprechend der Umgebungslufttemperatur und einer Temperatur der Heizplatte; und
Bestimmen der Heizleistung der Heizplatte durch die erste Steuereinheit in Abhängigkeit von der anfänglichen Heizleistung der Heizplatte und der Heizleistung der Heizplatte.

4. Das System nach einem der Ansprüche 2 bis 3, **dadurch gekennzeichnet, dass** die Art und Weise, wie das Heizungssteuerungsmodul der Heizungsleitung die Heizleistung der Heizungsleitung steuert, ist:

durch das Heizungssteuerungsmodul der Heizungsleitung, das einen Luftdurchsatz empfängt, der vom Ventilator eingegeben wird und von der ersten Steuereinheit in Echtzeit gesendet wird; und
durch das Heizungssteuerungsmodul der Heizungsleitung die Heizleistung der Heizungsleitung in Abhängigkeit von der durch den Ventilator eingegebenen Luftdurchflussmenge, der Temperatur des ersten Mischgasstroms, der Temperatur des zweiten Mischgasstroms und der vom Benutzer vorgegebenen Zieltemperatur zu bestimmen.

5. Das System nach Anspruch 4, **dadurch gekennzeichnet, dass** der Vorgang des Bestimmens der Heizleistung der Heizleitung durch die Steuereinheit der Heizung in Abhängigkeit von der vom Ventilator eingegebenen Luftmenge, der Temperatur des ersten Mischgasstroms, der Temperatur des zweiten Mischgasstroms und der vom Benutzer voreingestellten Zieltemperatur insbesondere aufweisend ist:

 durch das Heizungssteuerungsmodul der Heizungsleitung die Bestimmung einer anfänglichen Heizleistung der Heizungsleitung in Abhängigkeit von der durch den Ventilator eingegebenen Luftmenge, der Temperatur des ersten Mischgasstroms, der Temperatur des zweiten Mischgasstroms und der durch den Benutzer vorgegebenen Zieltemperatur;

 durch das Heizungssteuerungsmodul der Heizungsleitung, das die von der ersten Steuereinheit gesendete Umgebungslufttemperatur empfängt und entsprechend der Umgebungslufttemperatur und der Temperatur des ersten Mischgasstroms die Heizleistung der Heizungsleitung bestimmt; und

 durch das Heizungssteuerungsmodul der Heizungsrohrleitung die Heizleistung der Heizungsrohrleitung in Abhängigkeit von der Heizleistung der Heizungsrohrleitung und der anfänglichen Heizleistung der Heizungsrohrleitung bestimmt.

**6.** Das System nach einem der Ansprüche 1-5, **gekennzeichnet dadurch, dass** die erste Steuereinheit ein erstes Bestimmungsmodul aufweist, und das erste Bestimmungsmodul ist so konfiguriert, dass es, wenn die Wasserverdampfungsrate in dem Wassertank stabil bleibt, bestimmt, ob eine aktuelle Luftstromrate des Ventilators gleich der vom Benutzer voreingestellten Zielstromrate ist; und wenn die aktuelle Luftstromrate des Ventilators gleich der Zielstromrate ist, die erste Steuereinheit die Heizplatte so steuert, dass sie weiterhin mit einer aktuellen Heizleistung arbeitet, und gleichzeitig den Ventilator so steuert, dass er weiterhin mit der aktuellen Luftstromrate arbeitet.

**7.** Das System nach Anspruch 6, **dadurch gekennzeichnet, dass** die erste Steuereinheit weiterhin ein zweites Bestimmungsmodul aufweist, und das zweite Bestimmungsmodul so konfiguriert ist, dass es, wenn die aktuelle Luftstromrate des Ventilators kleiner als die Zielstromrate ist, bestimmt, ob die aktuelle Heizleistung der Heizplatte eine maximale Leistung ist und für ein zweites Zeitintervall aufrechterhalten wird, und wenn die aktuelle Heizleistung der Heizplatte die maximale Leistung ist und für das zweite Zeitintervall aufrechterhalten wird, die erste Steuereinheit die Heizplatte so steuert, dass sie mit der aktuellen Heizleistung weiterarbeitet, und den Ventilator so steuert, dass er gleichzeitig mit der aktuellen Luftstromrate weiterarbeitet.

**8.** Das System nach Anspruch 7, **dadurch gekennzeichnet, dass**, wenn das zweite Bestimmungsmodul feststellt, dass die aktuelle Heizleistung der Heizplatte nicht die maximale Leistung ist, die erste Steuereinheit die Heizleistung der Heizplatte neu bestimmt, und zwar zur gleichen Zeit, entsprechend einer Wasserverdampfungsrate der Verdampfung, wenn die Heizplatte mit der neu bestimmten Heizleistung der Heizplatte betrieben wird, die durch den Ventilator eingegebene Luftströmungsrate einstellt, bis die Wasserverdampfungsrate stabil bleibt, um die durch Mischen des Wasserdampfes und der Luft erhaltene Mischgasströmung in die Heizleitung einzugeben, wobei die relative Feuchtigkeit der Mischgasströmung die Zielfeuchtigkeit ist.

**9.** Ein Verfahren zur Steuerung der Befeuchtung einer Beatmungsvorrichtung, wobei das Verfahren auf ein Steuersystem zur Steuerung der Befeuchtung einer Beatmungsvorrichtung angewendet wird, das Steuersystem zur Steuerung der Befeuchtung einer Beatmungsvorrichtung eine Heizplatte, einen Wassertank, einen Ventilator und eine Heizleitung aufweist, und das Verfahren aufweist:

 Schritt S1: Empfangen einer Umgebungslufttemperatur, einer Umgebungsluftfeuchtigkeit und einer Zieltemperatur und einer Zielströmungsrate, die von einem Bediener vorgegeben werden, und Bestimmen einer Heizleistung der Heizplatte in Abhängigkeit von der Umgebungslufttemperatur, der Umgebungsluftfeuchtigkeit und der Zieltemperatur und der Zielströmungsrate, die vom Bediener vorgegeben werden, und Steuern der Heizplatte, um mit der Heizleistung zu arbeiten;

 Schritt S2: Bestimmen einer aktuellen Wasserverdampfungsrate in dem Wassertank;

 Schritt S3: gemäß der aktuellen Wasserverdampfungsrate in dem Wassertank, Bestimmen einer Luftströmungsrate, die durch den Ventilator eingegeben wird, so dass eine aktuelle relative Feuchtigkeit eines ersten gemischten Gasstroms, der durch Mischen eines Wasserdampfs in dem Wassertank und einer durch den Ventilator eingegebenen Luft erhalten wird, die Zielfeuchtigkeit ist;

 Schritt S4: gemäß einem dritten Zeitintervall, Überwachen einer Temperatur des ersten gemischten Gasstroms an einem Gaseinlass der Heizungsleitung und einer Temperatur eines zweiten gemischten Gasstroms an einem Gasauslass der Heizungsleitung;

 Schritt S5: Bestimmen einer aktuell erforderlichen Heizleistung der Heizleitung entsprechend der Temperatur des ersten gemischten Gasstroms und der Temperatur des zweiten gemischten Gasstroms, um die Heizleitung auf die aktuell erforderliche Heizleistung einzustellen, um eine Temperatur eines gemischten Gasstroms aufrechtzuerhalten, der durch Mischen von Wasserdampf, der über die Heizleitung zu der nasalen Sauerstoffkanüle geleitet wird, und Luft auf einer Zieltemperatur erhalten wird;

Schritt S6: Wiederholen der Ausführung von Schritt S2 bis Schritt S3, bis die Wasserverdampfungsrate in dem Wassertank stabil bleibt; und

Schritt S7: Wiederholung der Ausführung von Schritt S4, bis das Steuersystem für die Befeuchtung einer Vorrichtung zur Behandlung der Nasenschleimhaut den Betrieb anhält.

10. Das Verfahren nach Anspruch 9, **dadurch gekennzeichnet, dass** der Schritt S2 aufweist:
Überwachen einer Wassertank-Gastemperatur und einer Heizplattentemperatur an einem Wassertank-Gaseinlass gemäß einem ersten Zeitintervall, und Bestimmen der aktuellen Wasserverdampfungsrate in dem Wassertank gemäß der Wassertank-Gastemperatur und der Heizplattentemperatur.

11. Das Verfahren nach einem der Ansprüche 9-10, wobei der Schritt S1 aufweisend ist:

Bestimmen einer Gesamtwasserverdampfungsmenge in Abhängigkeit von der Soll-Durchflussrate, der Soll-Feuchtigkeit, der Umgebungsluft-Feuchtigkeit und der Umgebungsluft-Temperatur;

Bestimmen einer anfänglichen Heizleistung der Heizplatte entsprechend der Gesamtwasserverdunstungsmenge;

Bestimmen eines Heizwirkungsgrads der Heizplatte entsprechend der Umgebungslufttemperatur und einer Temperatur der Heizplatte; und

Bestimmen der Heizleistung der Heizplatte in Abhängigkeit von der anfänglichen Heizleistung der Heizplatte und der Heizleistung der Heizplatte.

12. Das Verfahren nach einem der Ansprüche 9-11, **dadurch gekennzeichnet, dass** der Schritt S4 aufweist:
Bestimmen der Temperatur des zweiten Mischgasstroms am Gasauslass der Heizungsrohrleitung entsprechend einer Wärmeabgaberate der Heizungsrohrleitung bei einer aktuellen Umgebungslufttemperatur und der Temperatur des ersten Mischgasstroms am Gaseinlass der Heizungsrohrleitung.

13. Das Verfahren nach einem der Ansprüche 9-12, **dadurch gekennzeichnet, dass** der Schritt S5 aufweisend ist:

Empfangen des vom Ventilator eingegebenen Luftdurchsatzes in Echtzeit;

Bestimmen einer anfänglichen Heizleistung der Heizleitung in Abhängigkeit von der durch den Ventilator eingegebenen Luftströmungsrate, der Temperatur des ersten Mischgasstroms, der Temperatur des zweiten Mischgasstroms und der durch den Benutzer voreingestellten Zieltemperatur;

Bestimmen der Heizleistung der Heizleitung in Abhängigkeit von der Umgebungslufttemperatur und der Temperatur des ersten Mischgasstroms; und

Bestimmen der Heizleistung der Heizungsrohrleitung entsprechend der Heizleistung der Heizungsrohrleitung und der anfänglichen Heizleistung der Heizungsrohrleitung.

14. Das Verfahren nach einem der Ansprüche 9-13, **dadurch gekennzeichnet, dass** das Verfahren nach dem Schritt S6 weiterhin aufweist:

wenn die Wasserverdampfungsrate im Wassertank stabil bleibt, Bestimmungsvorrichtung, ob eine aktuelle Luftstromrate des Ventilators gleich der vom Benutzer voreingestellten Zielstromrate ist;

wenn die aktuelle Luftströmungsrate des Ventilators gleich der Zielströmungsrate ist, Steuern der Heizplatte, um den Betrieb mit einer aktuellen Heizleistung fortzusetzen, und Steuern des Ventilators, um den Betrieb mit der aktuellen Luftströmungsrate fortzusetzen, zur gleichen Zeit;

wenn die aktuelle Luftdurchsatzrate des Ventilators kleiner als die Soll-Durchsatzrate ist, Bestimmen, ob die aktuelle Heizleistung der Heizplatte eine maximale Leistung ist und für ein zweites Zeitintervall aufrechterhalten wird, und wenn die aktuelle Heizleistung der Heizplatte die maximale Leistung ist und für das zweite Zeitintervall aufrechterhalten wird, Steuern der Heizplatte, um mit einer aktuellen Heizleistung weiterzuarbeiten, und Steuern des Ventilators, um gleichzeitig mit der aktuellen Luftdurchsatzrate weiterzuarbeiten; und

wenn festgestellt wird, dass die aktuelle Heizleistung der Heizplatte nicht die maximale Leistung ist, erneutes Bestimmen der Heizleistung der Heizplatte und gleichzeitig, entsprechend einer Wasserverdampfungsrate der Verdampfung, wenn die Heizplatte mit der neu bestimmten Heizleistung der Heizplatte arbeitet, Einstellen der durch den Ventilator eingegebenen Luftströmungsrate, bis die Wasserverdampfungsrate stabil bleibt, um die durch Mischen des Wasserdampfs und der Luft erhaltene Mischgasströmung in die Heizleitung einzugeben, wobei die relative Feuchtigkeit der Mischgasströmung die Zielfeuchtigkeit ist.

**Revendications**

1. Système de contrôle de l'humidification d'un appareil de traitement par ventilation, dans lequel

le système comprend un corps de dispositif de traitement par ventilation, un humidificateur de respiration, une conduite de chauffage et une canule nasale d'oxygène ;
le corps de dispositif de traitement par ventilation comprend un ventilateur et un premier dispositif de contrôle ;
l'humidificateur de respiration comprend un réservoir d'eau, une plaque chauffante, un capteur de température d'orifice d'entrée de gaz du réservoir d'eau, et un capteur de température de la plaque chauffante ; dans lequel un orifice de sortie de gaz du ventilateur est raccordé à un orifice d'entrée de gaz du réservoir d'eau ; un orifice de sortie de gaz du réservoir d'eau est raccordé à un orifice d'entrée de gaz de la conduite de chauffage ; un orifice de sortie de gaz de la conduite de chauffage est raccordé à une ouverture d'entrée de la canule nasale d'oxygène ; le capteur de température d'orifice d'entrée de gaz du réservoir d'eau est configuré pour mesurer une température de l'orifice d'entrée de gaz du réservoir d'eau ; le capteur de température de la plaque chauffante est configuré pour mesurer une température de la plaque chauffante ;
la conduite de chauffage comprend un capteur de température d'orifice d'entrée de gaz de la conduite de chauffage et un module de contrôle du réchauffement de la conduite de chauffage ; dans lequel le capteur de température d'orifice d'entrée de gaz de la conduite de chauffage est configuré pour mesurer une température de l'orifice d'entrée de gaz de la conduite de chauffage ;
le premier dispositif de contrôle est configuré pour recevoir une température de l'air ambiant et une humidité de l'air ambiant, une température du gaz du réservoir d'eau du capteur de température d'orifice d'entrée de gaz du réservoir d'eau, une température de la plaque chauffante transmise par le capteur de température de la plaque chauffante, et une humidité cible et un débit d'écoulement cible qui sont prédéfinis par un utilisateur, pour déterminer un débit d'écoulement de l'air entré par le ventilateur, de sorte qu'une humidité relative présente d'un premier écoulement de gaz mixte obtenue en mélangeant la vapeur d'eau dans le réservoir d'eau et l'air entré par le ventilateur est l'humidité cible ; et
le module de contrôle du réchauffement de la conduite de chauffage est configuré pour recevoir une température du premier écoulement de gaz mixte au niveau de l'orifice d'entrée de gaz de la conduite de chauffage et une température d'un second écoulement de gaz mixte au niveau de l'orifice de sortie de gaz, pour déterminer une puissance de chauffe actuellement requise de la conduite de chauffage, pour ajuster la conduite de chauffage à la puissance de chauffe actuellement requise, pour maintenir une température de l'écoulement de gaz mixte obtenue en mélangeant la vapeur d'eau transmise par l'intermédiaire de la conduite de chauffage vers la canule nasale d'oxygène et l'air à une température cible.

2. Système selon la revendication 1, **caractérisé en ce que**, le premier dispositif de contrôle comprend un module d'analyse du taux d'évaporation, un module de contrôle du débit d'écoulement et un module de contrôle du réchauffement et de l'humidification, et le module de contrôle du réchauffement et de l'humidification est configuré pour, en fonction du débit d'écoulement cible, l'humidité cible, de l'humidité de l'air ambiant et de la température de l'air ambiant, déterminer une puissance de chauffe de la plaque chauffante, et commander à la plaque chauffante de fonctionner à la puissance de chauffe ;

le module d'analyse du taux d'évaporation est configuré pour, en fonction d'un premier intervalle de temps, déterminer un taux présent d'évaporation de l'eau dans le réservoir d'eau en fonction de la température du gaz du réservoir d'eau et de la température de la plaque chauffante ; et
le module de contrôle du débit d'écoulement est configuré pour ajuster le débit d'écoulement de l'air entré par le ventilateur en fonction du taux présent d'évaporation de l'eau, de sorte qu'une humidité relative de l'écoulement de gaz mixte obtenue en mélangeant la vapeur d'eau et l'air est l'humidité cible, jusqu'à ce que le taux d'évaporation de l'eau dans le réservoir d'eau demeure stable.

3. Système selon l'une quelconque des revendications 1 à 2, **caractérisé en ce que**, le fonctionnement du premier dispositif de contrôle, en fonction du débit d'écoulement cible, de l'humidité cible, de l'humidité de l'air ambiant et de la température de l'air ambiant, en déterminant la puissance de chauffe de la plaque chauffante comprend particulièrement les étapes suivantes consistant à :

par le premier dispositif de contrôle, déterminer une quantité totale d'évaporation d'eau en fonction du débit d'écoulement cible, de l'humidité cible, de l'humidité de l'air ambiant et de la température de l'air ambiant ;

par le premier dispositif de contrôle, déterminer une puissance de chauffe initiale de la plaque chauffante en fonction de la quantité totale d'évaporation d'eau ;

par le premier dispositif de contrôle, déterminer une efficacité de chauffage de la plaque chauffante en fonction de la température de l'air ambiant et une température de la plaque chauffante ; et

par le premier dispositif de contrôle, déterminer la puissance de chauffe de la plaque chauffante en fonction de la puissance de chauffe initiale de la plaque chauffante et de l'efficacité de chauffage de la plaque chauffante.

4. Système selon l'une quelconque des revendications 2 à 3, **caractérisé en ce que**, la manière pour le module de contrôle du réchauffement de la conduite de chauffage de contrôler la puissance de chauffe de la conduite de chauffage est :

   par le module de contrôle du réchauffement de la conduite de chauffage, de recevoir un débit d'écoulement de l'air qui est entré par le ventilateur et qui est envoyé par le premier dispositif de contrôle en temps réel ; et
   par le module de contrôle du réchauffement de la conduite de chauffage, de déterminer la puissance de chauffe de la conduite de chauffage en fonction du débit d'écoulement de l'air entré par le ventilateur, de la température du premier écoulement de gaz mixte, de la température du second écoulement de gaz mixte et de la température cible prédéfinis par l'utilisateur.

5. Système selon la revendication 4, **caractérisé en ce que**, le fonctionnement, par le module de contrôle du réchauffement de la conduite de chauffage, en déterminant la puissance de chauffe de la conduite de chauffage en fonction du débit d'écoulement de l'air entré par le ventilateur, de la température du premier écoulement de gaz mixte, de la température du second écoulement de gaz mixte et de la température cible prédéfinis par l'utilisateur comprend particulièrement les étapes consistant à :

   par le module de contrôle du réchauffement de la conduite de chauffage, déterminer une puissance de chauffe initiale de la conduite de chauffage en fonction du débit d'écoulement de l'air entré par le ventilateur, la température du premier écoulement de gaz mixte, la température du second écoulement de gaz mixte et la température cible prédéfinis par l'utilisateur ;
   par le module de contrôle du réchauffement de la conduite de chauffage, recevoir la température de l'air ambiant envoyé par le premier dispositif de contrôle, et en fonction de la température de l'air ambiant et de la température du premier écoulement de gaz mixte, déterminer l'efficacité de chauffage de la conduite de chauffage ; et

par le module de contrôle du réchauffement de la conduite de chauffage, déterminer la puissance de chauffe de la conduite de chauffage en fonction de l'efficacité de chauffage de la conduite de chauffage et la puissance de chauffe initiale de la conduite de chauffage.

6. Système selon l'une quelconque des revendications 1 à 5, **caractérisé en ce que**, le premier dispositif de contrôle comprend un premier module de détermination, et le premier module de détermination est configuré pour, lorsque le taux d'évaporation de l'eau dans le réservoir d'eau demeure stable, déterminer si un débit présent d'écoulement de l'air du ventilateur est égal au débit d'écoulement cible prédéfini par l'utilisateur ; et

   lorsque le débit présent d'écoulement de l'air du ventilateur est égal au débit d'écoulement cible, le premier dispositif de contrôle commandant à la plaque chauffante de poursuivre le fonctionnement à la puissance de chauffe présente, et commandant au ventilateur de poursuivre le fonctionnement au débit présent d'écoulement de l'air en même temps.

7. Système selon la revendication 6, **caractérisé en ce que**, le premier dispositif de contrôle comprend en outre un second module de détermination, et le second module de détermination est configuré pour, lorsque le débit présent d'écoulement de l'air du ventilateur est inférieur au débit d'écoulement cible, déterminer si la puissance de chauffe présente de la plaque chauffante est une puissance maximale et étant maintenue durant un second intervalle de temps, et lorsque la puissance de chauffe présente de la plaque chauffante est la puissance maximale et étant maintenue durant le second intervalle de temps, le premier dispositif de contrôle commandant à la plaque chauffante de poursuivre le fonctionnement à la puissance de chauffe présente, et commandant au ventilateur de poursuivre le fonctionnement au débit présent d'écoulement de l'air en même temps.

8. Système selon la revendication 7, **caractérisé en ce que**, lorsque le second module de détermination détermine que la puissance de chauffe présente de la plaque chauffante n'est pas la puissance maximale, le premier dispositif de contrôle détermine à nouveau la puissance de chauffe de la plaque chauffante, et en même temps, en fonction d'un taux d'évaporation de l'eau de l'évaporation lorsque la plaque chauffante fonctionne à la puissance de chauffe à nouveau déterminée de la plaque chauffante, ajuster le débit d'écoulement de l'air entré par le ventilateur, jusqu'à ce que le taux d'évaporation de l'eau demeure stable, pour entrer l'écoulement de gaz mixte obtenu par mélange de la vapeur d'eau et de l'air dans la conduite de chauffage, dans lequel l'humidité relative de l'écoulement de gaz mixte est l'humidité cible.

9. Procédé de contrôle de l'humidification d'un appareil de traitement par ventilation, dans lequel le procédé est appliqué à un système de contrôle de l'humidification d'un appareil de traitement par ventilation, le système de contrôle de l'humidification d'un appareil de traitement par ventilation comprend une plaque chauffante, un réservoir d'eau, un ventilateur et une conduite de chauffage, et le procédé comprend :

étape S1: recevoir une température de l'air ambiant, une humidité de l'air ambiant et une température cible et un débit d'écoulement cible qui sont prédéfinis par un utilisateur, et en fonction de la température de l'air ambiant, de l'humidité de l'air ambiant et de la température cible et du débit d'écoulement cible qui sont prédéfinis par l'utilisateur, déterminer une puissance de chauffe de la plaque chauffante, et commander à la plaque chauffante de fonctionner à la puissance de chauffe ;

étape S2 : déterminer un taux présent d'évaporation de l'eau dans le réservoir d'eau ;

étape S3 : en fonction du taux présent d'évaporation de l'eau dans le réservoir d'eau, déterminer un débit d'écoulement de l'air entré par le ventilateur, de sorte qu'une humidité relative présente d'un premier écoulement de gaz mixte obtenue en mélangeant une vapeur d'eau dans le réservoir d'eau et l'air entré par le ventilateur est l'humidité cible ;

étape S4 : en fonction d'un troisième intervalle de temps, surveiller une température du premier écoulement de gaz mixte au niveau d'un orifice d'entrée de gaz de la conduite de chauffage et une température d'un second écoulement de gaz mixte au niveau d'un orifice de sortie de gaz de la conduite de chauffage ;

étape S5 : en fonction de la température du premier écoulement de gaz mixte et de la température du second écoulement de gaz mixte, déterminer une puissance de chauffe actuellement requise de la conduite de chauffage, pour ajuster la conduite de chauffage à la puissance de chauffe actuellement requise, pour maintenir une température d'un écoulement de gaz mixte obtenue en mélangeant la vapeur d'eau transmise par l'intermédiaire de la conduite de chauffage à la canule nasale d'oxygène et l'air à une température cible ;

étape S6 : répéter l'exécution de l'étape S2 à l'étape S3, jusqu'à ce que le taux d'évaporation de l'eau dans le réservoir d'eau demeure stable ; et

étape S7 : répéter l'exécution de l'étape S4, jusqu'à ce que le système de contrôle de l'humidification d'un appareil de traitement par ventilation arrête le fonctionnement.

10. Procédé selon la revendication 9, **caractérisé en ce que**, l'étape S2 comprend :
en fonction d'un premier intervalle de temps, surveiller une température du gaz du réservoir d'eau et une température de la plaque chauffante au niveau d'un orifice d'entrée de gaz du réservoir d'eau, et en fonction de la température du gaz du réservoir d'eau et de la température de la plaque chauffante, déterminer le taux présent d'évaporation de l'eau dans le réservoir d'eau.

11. Procédé selon l'une quelconque des revendications 9 à 10, dans lequel l'étape S1 comprend les étapes consistant à :

déterminer une quantité totale d'évaporation d'eau en fonction du débit d'écoulement cible, de l'humidité cible, de l'humidité de l'air ambiant et de la température de l'air ambiant ;
déterminer une puissance de chauffe initiale de la plaque chauffante en fonction de la quantité totale d'évaporation d'eau ;
déterminer une efficacité de chauffage de la plaque chauffante en fonction de la température de l'air ambiant et une température de la plaque chauffante ; et
déterminer la puissance de chauffe de la plaque chauffante en fonction de la puissance de chauffe initiale de la plaque chauffante et de l'efficacité de chauffage de la plaque chauffante.

12. Procédé selon l'une quelconque des revendications 9 à 11, **caractérisé en ce que**, l'étape S4 comprend l'étape consistant à:
en fonction d'un taux de dissipation de la chaleur de la conduite de chauffage à une température présente de l'air ambiant et la température du premier écoulement de gaz mixte au niveau de l'orifice d'entrée de gaz de la conduite de chauffage, déterminer la température du second écoulement de gaz mixte au niveau de l'orifice de sortie de gaz de la conduite de chauffage.

13. Procédé selon l'une quelconque des revendications 9 à 12, **caractérisé en ce que**, l'étape S5 comprend les étapes consistant à :

recevoir le débit d'écoulement de l'air entré par le ventilateur en temps réel ;

déterminer une puissance de chauffe initiale de la conduite de chauffage, en fonction du débit d'écoulement de l'air entré par le ventilateur, de la température du premier écoulement de gaz mixte, de la température du second écoulement de gaz mixte et de la température cible prédéfinis par l'utilisateur ;

déterminer l'efficacité de chauffage de la conduite de chauffage en fonction de la température de l'air ambiant et de la température du premier écoulement de gaz mixte ; et

déterminer la puissance de chauffe de la conduite de chauffage en fonction de l'efficacité de chauffage de la conduite de chauffage et de la puissance de chauffe initiale de la conduite de chauffage.

14. Procédé selon l'une quelconque des revendications 9 à 13, **caractérisé en ce que**, après l'étape S6, le procédé comprend en outre :

lorsque le taux d'évaporation de l'eau dans le réservoir d'eau demeure stable, déterminer si un débit présent d'écoulement de l'air du ventilateur est égal au débit d'écoulement cible prédéfini par l'utilisateur ;

lorsque le débit présent d'écoulement de l'air du ventilateur est égal au débit d'écoulement cible, commander à la plaque chauffante de poursuivre le fonctionnement à une puissance de chauffe présente, et commander au ventilateur de poursuivre le fonctionnement au débit présent d'écoulement de l'air en même temps ;

lorsque le débit présent d'écoulement de l'air du ventilateur est inférieur au débit d'écoulement cible, déterminer si la puissance de chauffe présente de la plaque chauffante est une puissance maximale et étant maintenue pour un second intervalle de temps, et lorsque la puissance de chauffe présente de la plaque chauffante est la puissance maximale et étant maintenue pour le second intervalle de temps, commander à la plaque chauffante de poursuivre le fonctionnement à une puissance de chauffe présente, et commander au ventilateur de poursuivre le fonctionnement au débit présent d'écoulement de l'air en même temps ; et

lors de la détermination que la puissance de chauffe présente de la plaque chauffante n'est pas la puissance maximale, déterminer à nouveau la puissance de chauffe de la plaque chauffante, et en même temps, en fonction d'un taux d'évaporation de l'eau de l'évaporation lorsque la plaque chauffante fonctionne à la puissance de chauffe à nouveau déterminée de la plaque chauffante, ajuster le débit d'écoulement de l'air entré par le ventilateur, jusqu'à ce que le taux d'évaporation de l'eau demeure stable, pour entrer l'écoulement de gaz mixte obtenu en mélangeant la vapeur d'eau et l'air dans la conduite de chauffage, dans lequel l'humidité relative de l'écoulement de gaz mixte est l'humidité cible.

FIG. 1

variation of evaporation rate of humidifier

water evaporation amount in 5 minutes (g)

operation period of time (minute)

FIG. 2

variation of heating-plate temperature of humidifier

heating-plate temperature of humidifier (°C)

operation period of time (minute)

FIG. 3

setting target flow rate, target
temperature and target humidity,
and beginning to operate

↓

detecting temperatures of
various points

↓

calculating required heating
power

↓

estimating water evaporation rate
according to temperature difference

↓

determining air flow rate
required to be inputted by fan
according to estimated water
evaporation rate

↓

whether air flow rate
reaches target flow rate

no →

whether reaches
maximum power and
maintains for second time
interval

no

yes ↓                    yes ↓

ending pre-heating, and
outputting at target flow rate

ending pre-heating, and
outputting at current fan flow
rate

FIG. 4

receiving an ambient-air temperature, an ambient-air humidity and a target temperature and a target flow rate that are preset by a user, and according to the ambient-air temperature, the ambient-air humidity and the target temperature and the target flow rate that are preset by the user, determining a heating power of the heating plate, and controlling the heating plate to operate at the heating power

S1

↓

determining a current water evaporation rate in the water tank

S2

↓

according to the current water evaporation rate in the water tank, determining an air flow rate inputted by the fan, so that a current relative humidity of a first mixed gas flow obtained by mixing a water vapor in the water tank and an air inputted by the fan is the target humidity

S3

↓

according to a third time interval, monitoring a temperature of the first mixed gas flow at a gas inlet of the heating pipeline and a temperature of a second mixed gas flow at a gas outlet of the heating pipeline

S4

↓

according to the temperature of the first mixed gas flow and the temperature of the second mixed gas flow, determining a currently required heating power of the heating pipeline, to adjust the heating pipeline to the currently required heating power, to in turn maintain a temperature of a mixed gas flow obtained by mixing a water vapor transmitted via the heating pipeline to the nasal oxygen cannula and an air at a target temperature

S5

↓

repeating to execute the Step S2 to the Step S3, till the water evaporation rate in the water tank maintains stable

S6

↓

repeating to execute the Step S4, till the humidification control system of a ventilation-treatment apparatus stops operating

S7

FIG. 5

EP 4 085 960 B1

calculating and processing device

memory 1020

processor 1010

space 1030 for the program code

program code 1031 for implementing the steps of
the method according to the present disclosure

FIG. 6

storage unit for the program code

readable code 1031' for implementing the steps
of the method according to the present disclosure

FIG. 7

28

**REFERENCES CITED IN THE DESCRIPTION**

**Patent documents cited in the description**

- US 2015217079 A1
- US 2015165146 A1
- US 2016151599 A1
- EP 4085960 A1